# EUROPÄISCHE PATENTANMELDUNG

(11) **EP 4 585 254 A1**
(43) Veröffentlichungstag der Anmeldung: **16.07.2025**
(21) Anmeldenummer: 24150859.7
(22) Anmeldetag: 09.01.2024
(51) Int. Cl.: A61M 35/00, A61K 9/00, A61L 2/16, A61M 5/31

(54) **WUNDSPÜLLÖSUNG ZUR ENTFERNUNG VON BIOFILMEN**

(71) Anmelder: Heraeus Medical GmbH, 61273 Wehrheim (DE)
(72) Erfinder: Dr. Vogt, Sebastian, 61273 Wehrheim (DE); Dr. Kluge, Thomas, 61273 Wehrheim (DE)
(74) Vertreter: Heraeus IP

(57) **Zusammenfassung**

Die vorliegende Anmeldung betrifft eine Vorrichtung (100) zur Herstellung einer antiseptischen Wundspüllösung, aufweisend ein antiseptisches Oxidationsmittel (104) und ein Tensid (124). Ebenfalls werden Verfahren zur Herstellung einer antiseptischen Wundspüllösung beschrieben. Solche Wundspüllösungen können insbesondere zur Behandlung von Biofilmen eingesetzt werden. Bevorzugt sind das antiseptische Oxidationsmittel und das Tensid jeweils als Feststoff und getrennt voneinander in der Vorrichtung angeordnet. Die Vorrichtung kann auch die Abgabe einer Wundspüllösung über eine Düse oder mittels Jet-Lavage-System ermöglichen.

## Beschreibung

### GEBIET DER ERFINDUNG

Die vorliegende Erfindung betrifft das Gebiet der Medizintechnik, insbesondere Vorrichtungen zur Präparation und Abgabe von Wirkstofflösungen, beispielsweise Wundspüllösungen.

### TECHNISCHER HINTERGRUND

Antiseptische Wirkstoffe und antiseptische Lösungen sind seit dem 19. Jahrhundert bekannt. Ein Beispiel für einen solchen Wirkstoff sind Hypochlorit-Salze und deren Lösungen. Die Haltbarkeit von wässrigen Hypochlorit-Lösungen ist beschränkt. Ursächlich hierfür ist hauptsächlich die langsam voranschreitende Umsetzung der Hypochlorit-Ionen zu Chloratlonen. Das bedeutet, dass der Hypochlorit-Gehalt der Lösungen mit zunehmender Lagerungszeit abnimmt. Daneben ist auch ein Zerfall von Hypochlorit-Ionen zu Chlorid-Ionen und Sauerstoff möglich.

Antiseptische Hypochlorit-Lösungen sind im Stand der Technik bekannt, wobei verschiedene Lösungen zur Verbesserung der Lagerungsstabilität vorgeschlagen worden sind.

Solche Lösungen wurden beispielsweise in den nachfolgenden Veröffentlichungen beschrieben: US8945630B2, US2011/0236490A1, WO2021001789, US2019328776A1, WO2021134041A1, WO2022/038507A1, WO2020252433, WO2020174436A1, US243781, US3749672, US6162371, WO1991/003936, US6471974, US2009/0258083, WO2010148004, WO2010148004, US2009/0148342A1, WO2016/100543A2, WO2020/089693A1, und WO2021/162736A1.

Allerdings ist ein vollständiger Schutz vor Zersetzung der Hypochlorit-Ionen in wässriger Lösung weiterhin herausfordernd.

Eine weitere Herausforderung ist die Bereitstellung von solchen Lösungen in steriler Form, wobei angestrebt wird, dass auch die Behältnisse steril und stabil sein sollen. Mit dieser Aufgabe beschäftigen sich beispielsweise US4964261A und US2005232848A1.

Feste Vorstufen zur Herstellung von Hypochlorit-haltigen Lösungen sind in DE2712574A1 und US4104190A beschrieben.

Eine besondere Herausforderung für Antiseptika stellen Biofilme dar. Diese bestehen je nach der Art der verursachenden Mikroorganismen aus einem Schleim aus Polysacchariden, Proteinen und eDNA. Dieser Film aus Schleim umgibt die darin angesiedelten Mikroorganismen und schützt diese gegenüber äußeren Einwirkungen. Auch die Zugänglichkeit der eingebetteten Mikroorganismen gegenüber Antiseptika ist vermindert.

### KURZE BESCHREIBUNG DER ERFINDUNG

Eine Aufgabe der vorliegenden Erfindung besteht darin, eines oder mehrere der oben geschilderten und weitere Probleme des Stands der Technik zu lösen. Beispielsweise liefert die Erfindung eine wirkstoffhaltige Vorrichtung, mit dessen Hilfe sich eine gebrauchsfertige Wirkstofflösung mithilfe handelsüblicher Behälter mit steriler Kochsalzlösung oder vergleichbaren Lösungen schnell, einfach und kostengünstig herstellen lässt. Da die erfindungsgemäße Vorrichtung den Wirkstoff bevorzugt in fester Form enthält, kann die Vorrichtung längere Zeit gelagert werden, auch wenn sie einen Wirkstoff enthält, der in wässriger Lösung nicht dauerhaft stabil ist. Zudem entfällt bei der Herstellung der erfindungsgemäßen Vorrichtung die Sterilisation von Flüssigkeiten, wenn die enthaltenen Stoffe darin in fester Form bereitgestellt sind. Einige Wirkstoffe lassen sich in fester Form besser sterilisieren, da sie sich in Lösung während der Sterilisierung leichter zersetzen. In einigen Ausführungsformen liefert die erfindungsgemäße Vorrichtung die Möglichkeit, die darin hergestellte Flüssigkeit direkt an einen Patienten abzugeben, beispielsweise mithilfe einer Düse, die an der erfindungsgemäßen Vorrichtung angebracht werden kann.

Der vorliegende Erfindung liegt insbesondere die Verbesserung der Zugänglichkeit von Mikroorganismen in Biofilmen durch die Verwendung von Tensiden zugrunde. Tenside können Biofilme lockern und auch ablösen. Eine derart verbesserte antiseptische Wundspüllösung kann ein Antiseptikum wie beispielsweise Hypochlorit-Lösungen oder auch Wasserstoffperoxidhaltige Lösungen aufweisen, die zusätzlich Tenside enthalten. Allerdings stellen wässrige Hypochlorit-Lösungen, wässrige Chlorit-Lösungen und auch Wasserstoffperoxid-Lösungen sehr starke Oxidationsmittel dar und können gelöste Tenside oxidativ angreifen. Dadurch ist die Lagerfähigkeit solcher Lösungen herausfordernd. Durch eine getrennte Anordnung der Substanzen kann deren Lagerfähigkeit verbessert werden.

Daher werden hierin Vorrichtungen beschrieben, mit deren Hilfe man intraoperativ oxidierende antiseptische Lösungen herstellen kann, welche Tenside enthalten, und bei der die bekannte Zersetzung von wässrigen Lösungen von Hypochlorit-Ionen, Chlorit-Ionen und auch von Wasserstoffperoxid während der Lagerung der Lösungen wirksam vermieden werden kann. Eine mit der Vorrichtung herstellbare wässrige Lösung kann Hypochlorit-Ionen und/oder Wasserstoffperoxid und/oder Chlorit-Ionen und mindestens ein Tensid enthalten. Weiterhin können die hergestellten Lösungen einen physiologisch akzeptablen pH-Wert aufweisen. Mithilfe der hierin beschriebenen Vorrichtungen kann eine Zersetzung des Tensids während der Lagerung der Vorrichtung wirksam verhindert werden. Die Vorrichtung kann gemeinsam mit den darin enthaltenen oxidierend wirkenden Antiseptika und dem Tensid sterilisierbar sein.

Bevorzugte Ausführungsformen der Erfindung werden nachstehend beschrieben.

Eine erste Ausführungsform betrifft eine Vorrichtung zur Herstellung einer antiseptischen Wundspüllösung, aufweisend ein antiseptisches Oxidationsmittel und ein Tensid. Eine zweite Ausführungsform betrifft eine Vorrichtung gemäß der ersten Ausführungsform, wobei das antiseptische Oxidationsmittel und das Tensid in der Vorrichtung getrennt voneinander vorliegen. Eine dritte Ausführungsform betrifft eine Vorrichtung nach einer der vorangehenden Ausführungsformen, wobei das antiseptische Oxidationsmittel und/oder das Tensid als Feststoff vorliegt. Eine vierte Ausführungsform betrifft eine Vorrichtung nach einer der vorangehenden Ausführungsformen, welche weiterhin ein erstes Reservoir und ein davon getrenntes zweites Reservoir aufweist, wobei das antiseptische Oxidationsmittel in dem ersten Reservoir angeordnet ist, und das Tensid in dem zweiten Reservoir angeordnet ist. Eine fünfte Ausführungsform betrifft eine Vorrichtung nach einer der vorangehenden Ausführungsformen, wobei die Vorrichtung weiterhin einen Zwischenfilter aufweist, der das erste Reservoir fluidleitend mit dem zweiten Reservoir verbindet, wobei der Zwischenfilter bevorzugt für das antiseptische Oxidationsmittel und/oder das Tensid nur in flüssigem Zustand durchlässig ist. Eine sechste Ausführungsform betrifft eine Vorrichtung nach einer der vorangehenden Ausführungsformen, wobei die Vorrichtung weiterhin ein Anschlusselement aufweist, wobei das Anschlusselement eingerichtet ist, eine fluidleitende Verbindung mit einem Behälter herzustellen. Eine siebte Ausführungsform betrifft eine Vorrichtung gemäß der sechsten Ausführungsform, wobei das antiseptische Oxidationsmittel und/oder das Tensid als Feststoff vorliegt, und wobei das Anschlusselement einen Anschlussfilter aufweist, welcher eingerichtet ist, das antiseptische Oxidationsmittel und/oder das Tensid als Feststoff in dem ersten Reservoir und/oder dem zweiten Reservoir zurückzuhalten. Eine achte Ausführungsform betrifft eine Vorrichtung gemäß der sechsten oder siebten Ausführungsform, weiterhin aufweisend ein Abgabeelement, wobei das Abgabeelement bevorzugt eingerichtet ist, unabhängig von dem Anschlusselement eine aus dem antiseptischen Oxidationsmittel und dem Tensid hergestellte desinfizierende Wundspüllösung aus der Vorrichtung abzugeben. Eine neunte Ausführungsform betrifft eine Vorrichtung nach einer der vorangehenden Ausführungsformen, welche weiterhin einen Dorn aufweist, der zum Durstechen eines Septums eingerichtet ist, wobei der Dorn bevorzugt einen hohlen Innenraum aufweist, der zur Aufnahme einer Flüssigkeit aus einem Behälter in das erste Reservoir und das zweite Reservoir eingerichtet ist. Eine zehnte Ausführungsform betrifft eine Vorrichtung nach einer der vorangehenden Ausführungsformen, welche weiterhin ein Fluidikelement aufweist, das eingerichtet und ausgebildet ist, eine Flüssigkeit in die Vorrichtung zu fördern. Eine elfte Ausführungsform betrifft eine Vorrichtung nach einer der Ausführungsformen vier bis zehn, wobei die Vorrichtung ein Adapterteil und ein Mischerteil aufweist, wobei das Adapterteil zum Anschluss an einen Behälter eingerichtet ist, und das Mischerteil zum Mischen des antiseptischen Oxidationsmittels und des Tensids mit einer Flüssigkeit aus dem Behälter eingerichtet ist. Eine zwölfte Ausführungsform betrifft eine Vorrichtung nach einer der vorangehenden Ausführungsformen, wobei das antiseptische Oxidationsmittel ausgewählt ist aus der Gruppe bestehend aus Chlorit, Hypochlorit und Peroxid, weiter bevorzugt ausgewählt aus der Gruppe bestehend aus Calciumhypochlorit, Magnesiumhypochlorit, Natriumchlorit, Natriumhypochlorit, Natriumperoxid und Calciumperoxid. Eine dreizehnte Ausführungsform betrifft eine Vorrichtung nach einer der vorangehenden Ausführungsformen, wobei das Tensid ein Fettalkoholsulfat und/oder ein quartäres Ammoniumsalz umfasst; wobei das Tensid weiter bevorzugt Natriumdodecylsulfat und/oder Benzalkoniumchlorid umfasst. Eine vierzehnte Ausführungsform betrifft eine Vorrichtung nach einer der vorangehenden Ausführungsformen, welche weiterhin einen pH-Regulator aufweist, wobei der pH-Regulator ausgewählt ist aus der Gruppe bestehend aus Citronensäure, Citrat, Glucuronsäure, Gluconat, Natriumhydrogensulfat, und Natriumhydrogencarbonat.

Ein zweiter Aspekt betrefft in einer ersten Ausführungsform ein Verfahren zur Herstellung einer antiseptischen Wundspüllösung mithilfe einer Vorrichtung nach einem der vorangehenden Ansprüche, wobei das Verfahren die folgenden Schritte aufweist,
a) gegebenenfalls Auflösen oder Verdünnen des antiseptischen Oxidationsmittel und/oder des Tensid mithilfe einer medizinisch verträglichen Flüssigkeit mithilfe der Vorrichtung,
b) Mischen des antiseptischen Oxidationsmittel mit dem Tensid mithilfe der Vorrichtung, und dadurch Erhalt einer antiseptischen Wundspüllösung. In einer zweiten Ausführungsform des Verfahrens wird ein Verfahren nach der ersten Ausführungsform bereitgestellt, wobei die Konzentration des antiseptischen Oxidationsmittels in der hergestellten Wundspüllösung 100 ppm bis 4000 ppm (Masse/Masse) beträgt, und/oder die Konzentration des Tensids in der hergestellten Wundspüllösung 25 ppm bis 2000 ppm (Masse/Masse) beträgt. in einer dritten Ausführungsform des Verfahrens wird ein Verfahren nach der ersten oder zweiten Ausführungsform bereitgestellt, wobei der pH-Wert der hergestellten Wundspüllösung 4 bis 8, bevorzugt 5 bis 7 beträgt.

In einem dritten Aspekt wird in einer erster Ausführungsform eine Vorrichtung zur Herstellung einer Wirkstofflösung, bevorzugt einer antiseptischen Wundspüllösung, beschrieben, aufweisend
- ein Anschlusselement, das zum flüssigkeitsdichten Anschluss an einen Behälter ausgebildet und eingerichtet ist;
- einen Dorn, der zum Durchstechen eines Septums eines Behälters ausgebildet und eingerichtet ist,
- ein erstes Reservoir, das einen Wirkstoff, wie zum Beispiel ein antiseptisches Oxidationsmittel, in fester Form enthält, und über einen Ausgang des Reservoirs fluidleitend mit dem Anschlusselement verbindbar ist;
- einen Anschlussfilter, der an dem Ausgang des ersten Reservoirs angeordnet ist, und eingerichtet ist, den Wirkstoff in fester Form in dem Reservoir zurückzuhalten;
- einen Zwischenfilter, der das erste Reservoir mit einem zweiten Reservoir verbindet, wobei das zweite Reservoir ein Tensid enthält;
- ein Fluidikelement, welches zur Aufnahme einer Flüssigkeit aus einem Behälter in das erste und das zweite Reservoir eingerichtet ist, und/oder zur Abgabe einer Flüssigkeit aus dem ersten und dem zweiten Reservoir eingerichtet ist;
wobei die Vorrichtung ausgebildet und eingerichtet ist, eine Flüssigkeit aus einem Behälter in das erste und das zweite Reservoir aufzunehmen, den Wirkstoff und das Tensid in der Flüssigkeit zu lösen, und den gelösten Wirkstoff und das gelöste Tensid über das Anschlusselement an einen Patienten abzugeben.

Eine zweite Ausführungsform des dritten Aspekts betrifft eine Vorrichtung nach Ausführungsform 1, wobei der Dorn einen hohlen Innenraum aufweist, der zur Aufnahme einer Flüssigkeit aus einem Behälter in das erste Reservoir eingerichtet ist.

Eine dritte Ausführungsform des dritten Aspekts betrifft eine Vorrichtung nach einer der vorangehenden Ausführungsformen, wobei das Fluidikelement einen innerhalb des ersten und/oder zweiten Reservoirs beweglich angeordneten Kolben aufweist, wobei der Kolben bevorzugt das erste und/oder zweite Reservoir flüssigkeitsdicht abschließt.

Eine vierte Ausführungsform des dritten Aspekts betrifft eine Vorrichtung nach einer der vorangehenden Ausführungsformen, welche ein Adapterteil und ein Mischerteil aufweist, welche lösbar miteinander verbindbar sind.

Eine fünfte Ausführungsform des dritten Aspekts betrifft eine Vorrichtung nach Ausführungsform 4, wobei das Adapterteil das Anschlusselement und den Dorn enthält, und/oder wobei das Mischerteil das Reservoir, den Filter und das Fluidikelement enthält.

Eine sechste Ausführungsform des dritten Aspekts betrifft eine Vorrichtung nach Ausführungsform 4 oder 5, wobei die Vorrichtung weiterhin ein Gewinde aufweist, mit dessen Hilfe das Adapterteil mit dem Mischerteil verbindbar ist.

Eine siebte Ausführungsform des dritten Aspekts betrifft eine Vorrichtung nach einer der vorangehenden Ausführungsformen, wobei die Vorrichtung vollständig sterilisiert ist, bevorzugt vollständig pyrogenfrei ist.

Eine achte Ausführungsform des dritten Aspekts betrifft eine Vorrichtung nach einer der vorangehenden Ausführungsformen, wobei die Vorrichtung weiterhin eine Düse aufweist, welche zur Abgabe des Wirkstoffs in gelöster Form eingerichtet ist.

Eine neunte Ausführungsform des dritten Aspekts betrifft eine Vorrichtung gemäß Ausführungsform 8, wobei die Düse lösbar fluidleitend mit dem Anschlusselement verbindbar ist.

Eine zehnte Ausführungsform des dritten Aspekts betrifft eine Vorrichtung nach einer der vorangehenden Ausführungsformen, wobei der Wirkstoff ein Antiseptikum ist, welches bevorzugt ausgewählt ist aus der Gruppe bestehend aus Calciumhypochlorit, Magnesiumhypochlorit, Natriumhypochlorit, Natriumpercarbonat, Natriumperoxid, Calciumperoxid, Harnstoff-Peroxid, Triisocyanurchlorid, Natrium-N-chlor-4-methylbenzolsulfonamid, Natrium-N-chlorbenzolsulfonamid, Octenidin, Polyhexanid und Chlorhexidindigluconat.

Eine elfte Ausführungsform des dritten Aspekts betrifft eine Vorrichtung nach einer der vorangehenden Ausführungsformen, wobei das Anschlusselement eine erste Durchführung aufweist, welche zur Aufnahme einer Flüssigkeit aus einem Behälter in das erste Reservoir eingerichtet ist.

Eine zwölfte Ausführungsform des dritten Aspekts betrifft eine Vorrichtung nach einer der vorangehenden Ausführungsformen, wobei das Anschlusselement eine zweite Durchführung aufweist, welche zur Abgabe einer Flüssigkeit aus einem Behälter eingerichtet ist, wobei die Abgabe bevorzugt in einem Zustand möglich ist, in welchem die Vorrichtung über die erste Durchführung gemäß Ausführungsform 11 mit dem Reservoir verbunden ist.

Eine dreizehnte Ausführungsform des dritten Aspekts betrifft eine Vorrichtung nach einer der vorangehenden Ausführungsformen, wobei das erste Reservoir und das zweite Reservoir durchlässig für Gammastrahlen und/oder Elektronenstrahlen sind.

Ein vierter Aspekt betrifft ein Kit, aufweisend eine Vorrichtung nach einem der Ausführungsformen 1 bis 13 des dritten Aspekts, oder einer Ausführungsform des ersten Aspekts, und einen Behälter mit einer medizinisch verträglichen Flüssigkeit, wobei die Vorrichtung lösbar mit dem Behälter verbindbar ist, wobei das Kit gemeinsam keimdicht verpackt, bevorzugt vollständig sterilisiert, weiter bevorzugt vollständig pyrogenfrei ist.

Ein fünfter Aspekt betrifft einen Wirkstoff zur Verwendung in einem medizinischen Behandlungsverfahren, wobei der Wirkstoff mithilfe einer Vorrichtung nach einer der vorangegangenen Ausführungsformen unmittelbar vor der Verabreichung an einen Patienten in einer Flüssigkeit gelöst und anschließend in gelöster Form verabreicht wird.

Ein sechster Aspekt betrifft ein Verfahren zur Herstellung einer Wirkstofflösung, welches die nachfolgenden Schritte umfasst,
a) Aufnahme einer Flüssigkeit aus einem Behälter mithilfe einer Vorrichtung nach einer der Ausführungsformen 1 bis 13 des dritten Aspekts, oder einer Ausführungsform des ersten Aspekts, in das erste Reservoir und das zweite Reservoir der Vorrichtung,
b) Mischen des Wirkstoffs in dem ersten Reservoir und des Tensids in dem zweiten Reservoir mit der in Schritt a) aufgenommenen Flüssigkeit,
c) dadurch Erhalt einer Wirkstofflösung,
d) gegebenenfalls Abgabe der Wirkstofflösung in den Behälter.

Ein siebter Aspekt betrifft die Verwendung einer Vorrichtung nach einer der Ausführungsformen 1 bis 13 des dritten Aspekts, oder einer Ausführungsform des ersten Aspekts, zur Herstellung einer Wirkstofflösung.

Ein achter Aspekt betrifft eine medizinische Wundspüllösung zur Anwendung in einem Verfahren für die Prophylaxe oder Behandlung einer Wundinfektion, wobei die medizinische Wundspüllösung ein antiseptisches Oxidationsmittel und ein Tensid umfasst.

### KURZE BESCHREIBUNG DER ZEICHNUNGEN

**Figur 1** zeigt ein Mischerteil mit einem ersten Reservoir für einen Wirkstoff, einem zweiten Reservoir für ein Tensid, und einem Fluidikelement zum Transport einer Flüssigkeit aus einem Behälter.
**Figur 2** zeigt ein Adapterteil, welches mit einem Behälter verbindbar ist.
**Figur 3** zeigt ein Adapterteil in einem verschlossenen und versiegelten Zustand.
**Figur 4** zeigt einen Behälter mit einem Septum, der mit einer erfindungsgemäßen Vorrichtung verbunden werden kann.
**Figur 5** zeigt ein Adapterteil, das mit einem Behälter verbunden ist.
**Figur 6** zeigt eine erfindungsgemäße Vorrichtung, die mit einem Behälter verbunden ist.
**Figur 7** zeigt eine erfindungsgemäße Vorrichtung, die Flüssigkeit aus einem Behälter aufnimmt.
**Figur 8** zeigt eine erfindungsgemäße Vorrichtung, die eine darin gebildete Wundspüllösung an einen Behälter abgibt.
**Figur 9** zeigt eine erfindungsgemäße Vorrichtung, die eine darin hergestellte Wundspüllösung vollständig an einen Behälter abgegeben hat.
**Figur 10** zeigt einen Behälter, der mit einem Adapterteil und einer Düse versehen ist.
**Figur 11** zeigt ein Kit, welches einen Behälter, ein Adapterteil, ein Mischerteil, eine Düse und eine Abgabevorrichtung aufweist.

### AUSFÜHRLICHE BESCHREIBUNG

Zu den hierin beschriebenen Ausführungsformen, deren Elemente ein bestimmtes Merkmal (z.B. ein Material) "aufweisen", "enthalten", oder "umfassen" wird grundsätzlich immer eine weitere Ausführungsform erwogen, in denen das betreffende Element allein aus dem Merkmal besteht, d.h. keine weiteren Bestandteile umfasst. Das Wort "umfassen" oder "umfassend" wird hierin synonym mit dem Wort "enthalten", "enthaltend", "aufweisen" oder "aufweisend" verwendet.

Wenn in einer Ausführungsform ein Element mit dem Singular bezeichnet ist, wird ebenfalls eine Ausführungsform erwogen, bei denen mehrere dieser Elemente vorhanden sind. Die Verwendung eines Begriffs für ein Element im Plural umfasst grundsätzlich auch eine Ausführungsform, in welchem nur ein einzelnes entsprechendes Element enthalten ist.

Soweit nicht anders angegeben oder aus dem Zusammenhang eindeutig ausgeschlossen, ist es grundsätzlich möglich und wird hiermit eindeutig in Betracht gezogen, dass Merkmale unterschiedlicher Ausführungsformen auch in den anderen hierin beschriebenen Ausführungsformen vorhanden sein können. Ebenso wird grundsätzlich erwogen, dass alle Merkmale, die hierin in Zusammenhang mit einem Verfahren oder einem Stoff zur Anwendung in einem Verfahren beschrieben werden, auch für die hierin beschriebenen Erzeugnisse und Vorrichtungen anwendbar sind, und umgekehrt. Lediglich aus Gründen der knapperen Darstellung werden alle diese erwogenen Kombinationen nicht in allen Fällen explizit aufgeführt. Auch technische Lösungen, die zu den hierin beschriebenen Merkmalen bekanntermaßen gleichwertig sind, sollen grundsätzlich vom Umfang der Erfindung umfasst sein.

Eine erste Ausführungsform betrifft eine Vorrichtung zur Herstellung einer antiseptischen Wundspüllösung. Die Vorrichtung weist einen antiseptischen Wirkstoff, wie z.B. ein antiseptisches Oxidationsmittel, auf. Die Vorrichtung weist weiterhin ein Tensid auf.

Der Begriff "antiseptisch" bezeichnet eine bakterizide oder fungizide, d. h. keimtötende, Wirkung eines Stoffs, insbesondere eines Wirkstoffs, oder einer Zusammensetzung, wie z.B. einer Wundspüllösung. Eine Wundspüllösung ist eine wässrige Zusammensetzung, welche medizinisch verträglich ist und für intraoperative Spülungen, die auch als Lavage bezeichnet werden, geeignet ist.

Ein Tensid ist ein amphiphiler Stoff, welcher einen hydrophilen Molekülteil und einen hydrophoben Molekülteil aufweist, und dadurch oberflächenaktive Eigenschaften besitzt. Tenside verbessern dadurch die Löslichkeit hydrophober Substanzen in wässrigen Lösungen. Durch den Einsatz von Tensiden in Wundspüllösungen kann die Stabilität von Biofilmen herabgesetzt, und dadurch die Wirkung eines antiseptischen Oxidationsmittels auf den Biofilm verbessert werden. Beispiele für erfindungsgemäß verwendbare Tenside sind Fettalkoholsulfate und quartäre Ammoniumsalze. Bevorzugte quartäre Ammoniumsalze sind Chloride von quartären Ammoniumionen. Ein Beispiel für ein Fettalkoholsulfat ist Natriumdodecylsulfat, welches auch als Natriumlaurylsulfat oder SDS bezeichnet wird. Ein Beispiel für ein quartäres Ammoniumsalz ist Benzalkoniumchlorid. Das Tensid kann ein an anionisches, kationisches oder ein neutral geladenes Tensid sein, z.B. ein nichtionisches oder zwitterionisches Tensid. Bevorzugt sind Tenside, welche in wässrigen Lösungen von Calciumsalzen keine Calciumhaltigen Präzipitate bilden. Weiterhin bevorzugt sind Tenside, welche zur innerlichen Anwendung im Rahmen einer chirurgischen Operation medizinisch verträglich sind. In einer Ausführungsform umfasst das Tensid ein Fettalkoholsulfat. In einer Ausführungsform umfasst das Tensid ein quartäres Ammoniumsalz. In einer Ausführungsform umfasst das Tensid Natriumdodecylsulfat. In einer Ausführungsform umfasst das Tensid Benzalkoniumchlorid.

Vorteilhafterweise können das antiseptische Oxidationsmittel und das Tensid getrennt voneinander in der Vorrichtung vorliegen.

Das antiseptische Oxidationsmittel kann als Feststoff in der Vorrichtung vorliegen. Das Tensid kann als Feststoff in der Vorrichtung vorliegen. Bevorzugt liegen sowohl das antiseptische Oxidationsmittel als auch das Tensid jeweils als Feststoff in der Vorrichtung vor.

Bevorzugt ist das antiseptische Oxidationsmittel wasserlöslich. Bevorzugt ist das Tensid wasserlöslich. Besonders bevorzugt sind sowohl das antiseptische Oxidationsmittel als auch das Tensid jeweils wasserlöslich. Als "wasserlöslich" wird erfindungsgemäß eine Substanz verstanden, deren Löslichkeit in Wasser bei 25 °C und 1025 hPa mindestens 1 g Substanz pro Liter Wasser beträgt.

In einer Ausführungsform weist die Vorrichtung weiterhin einen pH-Regulator auf. Ein pH-Regulator ist eine Substanz, mit deren Hilfe die mit der Vorrichtung herstellbare Wundspüllösung auf einen medizinisch verträglichen pH-Wert gebracht werden kann. Ein medizinisch verträglicher pH-Wert beträgt pH 4 bis 8, bevorzugt 5 bis 7, wobei der pH-Wert bei 25°C und 1025 hPa gemessen wird. Der pH-Regulator ist bevorzugt eine Säure. In einigen Ausführungsformen ist der pH-Regulator ausgewählt aus der Gruppe bestehend aus Citronensäure, Citrat, Glucuronsäure, Gluconat, Natriumhydrogensulfat, und Natriumhydrogencarbonat. In einer Ausführungsform weist das erste Reservoir einen pH-Regulator auf. In einer Ausführungsform weist das zweite Reservoir einen pH-Regulator auf. In einer Ausführungsform weisen sowohl das erste Reservoir als auch das zweite Reservoir einen pH-Regulator auf. Der pH-Regulator kann als Feststoff in der Vorrichtung vorliegen. Der pH-Regulator kann als Mischung mit dem antiseptischen Oxidationsmittel und/oder dem Tensid vorliegen.

Leicht saure bis neutrale pH-Werte können insbesondere bei der Verwendung von Hypochlorit-haltigen und Peroxid-haltigen antiseptischen Oxidationsmitteln vorteilhaft sein, um ein erwünschtes Gleichgewicht von Hypochlorit-Ionen zu hypochloriger Säure bzw. Peroxid-Ionen zu Hydroperoxid-Ionen einzustellen. Hierdurch kann ein wirksamer antiseptischer Effekt der herzustellenden Wundspüllösung erreicht werden.

Die erfindungsgemäße Vorrichtung kann ein Adapterteil und ein Mischerteil aufweisen. Das Adapterteil kann mit dem Mischerteil verbindbar sein. Das Adapterteil kann mit dem Mischerteil lösbar verbindbar sein, bevorzugt formschlüssig verbindbar sein, z.B. mithilfe eines Gewindes oder einer Schnappverbindung.

Bevorzugt weist eine erfindungsgemäße Vorrichtung ein erstes Reservoir und ein davon getrenntes zweites Reservoir auf. Bevorzugt ist das antiseptische Oxidationsmittel in dem ersten Reservoir angeordnet, und das Tensid ist in dem zweiten Reservoir der Vorrichtung angeordnet. Das erste Reservoir und das zweite Reservoir können in dem Mischerteil angeordnet sein.

Die Vorrichtung kann einen Zwischenfilter aufweisen, der das erste Reservoir fluidleitend mit dem zweiten Reservoir verbindet. Der Zwischenfilter ist bevorzugt nur in flüssigem Zustand durchlässig für das antiseptische Oxidationsmittel und/oder das Tensid. Dies bedeutet, dass das antiseptische Oxidationsmittel bzw. das Tensid den Zwischenfilter nicht als Feststoff, sondern beispielsweise nur in gelöstem Zustand passieren können. Wenn beispielsweise das Oxidationsmittel und das Tensid jeweils als Feststoff in der Vorrichtung vorliegen, kann der Zwischenfilter demnach diese beiden Stoffe so lange voneinander getrennt halten, bis sie in einer Flüssigkeit, bevorzugt einer wässrigen Lösung, aufgelöst werden. Das Oxidationsmittel und das Tensid werden in diesem Fall erst durch ihre Lösung in einem wässrigen Lösungsmittel miteinander in Kontakt gebracht, um eine Wundspüllösung daraus herzustellen. Demnach sind Ausführungsformen der Vorrichtung mit Zwischenfilter vorteilhaft, wenn ein antiseptisches Oxidationsmittel und ein Tensid in fester Form, beispielsweise in Pulverform, in der Vorrichtung bereitgestellt werden. Der Zwischenfilter kann beweglich in der Vorrichtung angeordnet sein, beispielsweise verschiebbar sein, um eine möglichst vollständige Abgabe von Flüssigkeit aus der Vorrichtung zu ermöglichen. Bevorzugt ist der Zwischenfilter angeordnet und eingerichtet, so dass sich die Position des Zwischenfilters nicht in unerwünschter Weise selbstständig (z.B. während des Transports der Vorrichtung), sondern nur durch gezielte Einwirkung eines Benutzers, beispielsweise durch unmittelbare Ausübung einer Kraft auf den Zwischenfilter mithilfe eines Spritzenkolbens, verändern kann. Hierzu kann der Filter beispielsweise mit einer entsprechend angepassten Klemmspannung in einem zylindrischen Innenraum des Mischerteils der Vorrichtung angebracht sein, so dass er sich erst bei Überschreitung einer vorbestimmten Kraft auf den Filter bewegen kann.

Anstelle des Zwischenfilters kann eine Trennwand zwischen dem ersten und zweiten Reservoir angeordnet sein. Die Trennwand kann eingerichtet sein, das erste Reservoir flüssigkeitsdicht vom zweiten Reservoir abzugrenzen. Durch Entfernen oder Durchbrechen der Trennwand können die Inhalte des ersten und des zweiten Reservoirs, z.B. ein antiseptisches Oxidationsmittel und ein Tensid, miteinander gemischt werden. Auf diese Weise können in der Vorrichtung ein antiseptisches Oxidationsmittel und/oder ein Tensid in flüssiger Form, z.B. in wässriger Lösung, getrennt voneinander angeordnet werden.

Die Vorrichtung weist bevorzugt ein Anschlusselement auf. Das Anschlusselement kann in dem Adapterteil angeordnet sein. Das Anschlusselement ist bevorzugt zum flüssigkeitsdichten und fluidleitenden Anschluss der Vorrichtung an einen Behälter ausgebildet und eingerichtet. Hierbei kann das Anschlusselement wie nachfolgend beschrieben mit einem Dorn zusammenwirken. Dadurch kann Flüssigkeit zwischen der Vorrichtung und dem Behälter ausgetauscht werden, ohne dass Flüssigkeit nach außen dringt. Das Anschlusselement kann hierzu eine formschlüssige Verbindung zwischen Adapterteil und Behälter ermöglichen. Solche Behälter können beispielsweise Verpackungen sein, in denen physiologische Kochsalzlösungen, Infusionslösungen und ähnliche flüssige Medizinprodukte handelsüblich erhältlich sind. Gebräuchlich hierfür sind beispielsweise Flaschen oder Folienbeutel. Diese Behälter bestehen häufig aus Kunststoff. Der Behälter kann ein Behälter mit flexibler, bevorzugt elastisch verformbarer, Außenwand sein, sodass eine darin enthaltene Flüssigkeit durch Zusammendrücken des Behälters in einem geöffneten Zustand aus dem Behälter freigesetzt werden kann. Der Behälter kann ein Septum umfassen, welches mit einem an der Vorrichtung angebrachten Dorn durchstochen werden kann.

Das Anschlusselement der Vorrichtung kann bevorzugt formschlüssig mit einem Behälter verbindbar sein. Das Anschlusselement kann lösbar mit einem Behälter verbindbar sein. Beispielsweise kann das Anschlusselement an die Öffnung einer Kunststoffflasche oder den Anschluss eines Folienbeutels aufsteckbar sein. Das Anschlusselement kann ein Gewinde oder eine Schnappverbindung aufweisen.

Die Vorrichtung kann weiterhin einen Dorn aufweisen, der zum Durchstechen eines Septums eines Behälters ausgebildet und eingerichtet ist. Der Dorn kann hierzu eine spitz zulaufende Form aufweisen, mit dessen Spitze ein Septum eines Behälters durch manuelles Ausüben von Druck des Dorns auf das Septum durchdrungen werden kann. Vergleichbare Strukturen werden zum Anschluss von Infusionbestecken an Behältern mit Infusionslösungen verwendet. Der Dorn kann in dem Adapterteil angeordnet sein.

In einer Ausführungsform weist der Dorn einen hohlen Innenraum auf, der zur Aufnahme einer Flüssigkeit aus einem Behälter in das Reservoir eingerichtet ist. Hierbei kann der Dorn beispielsweise in der Form einer Hohlnadel ausgestaltet sein. Der Dorn kann beispielsweise aus Kunststoff bestehen, oder einen Kunststoff umfassen.

Bevorzugt ist der Dorn ausreichend steif, um ein Septum eines handelsüblichen Behälters für physiologische Kochsalzlösung zu durchdringen, jedoch nicht so hart und fest, dass eine Verletzungsgefahr für medizinisches Personal besteht. In einer Ausführungsform ist der Dorn keine Metallnadel.

Die Vorrichtung weist bevorzugt ein erstes Reservoir auf. Das erste Reservoir enthält bevorzugt einen Stoff in fester Form, beispielsweise als Pulver. Der Stoff kann ein antiseptisches Oxidationsmittel sein. Das erste Reservoir kann eine im wesentlichen zylindrische Form aufweisen, ähnlich dem Innenraum einer Spritze.

Die Vorrichtung kann eingerichtet sein, den enthaltenen Stoff, insbesondere ein antiseptisches Oxidationsmittel, in gelöster Form in den Behälter abzugeben.

Die Vorrichtung kann weiterhin ein zweites Reservoir aufweisen. Das zweite Reservoir kann einen Stoff enthalten, der sich von dem Stoff in dem ersten Reservoir unterscheidet. Das zweite Reservoir kann bevorzugt ein Tensid enthalten. Das zweite Reservoir kann eine im wesentlichen zylindrische Form aufweisen. In einer Ausführungsform weisen das erste und das zweite Reservoir jeweils eine zylindrische Form auf. In einer Ausführungsform sind das erste Reservoir und das zweite Reservoir entlang einer gemeinsamen Achse angeordnet.

In einer Ausführungsform ist ein antiseptisches Oxidationsmittel in dem ersten Reservoir angeordnet, und ein Tensid ist in dem zweiten Reservoir angeordnet. In einer Ausführungsform ist ein antiseptisches Oxidationsmittel in dem zweiten Reservoir angeordnet, und ein Tensid ist in dem ersten Reservoir angeordnet.

Die Vorrichtung weist bevorzugt einen Filter auf. Ein solcher Filter ist bevorzugt eingerichtet, einen festen Stoff, zum Beispiel ein antiseptisches Oxidationsmittel oder ein Tensid, in einem Reservoir, zum Beispiel dem ersten und/oder zweiten Reservoir der Vorrichtung, zurückzuhalten, solange der Stoff nicht in einer Flüssigkeit gelöst ist. Auf diese Weise kann verhindert werden, dass Stoff in ungelöster Form an einen Patienten abgegeben wird. Es ist erfindungsgemäß bevorzugt, dass der Stoff nur in flüssiger Form, z.B. in vollständig wässrig gelöster Form, an einen Patienten abgegeben werden kann. Hierzu kann der Filter bevorzugt flüssigkeitsdurchlässig sein. Der Filter ist bevorzugt gasdurchlässig. Ein gasdurchlässiger Filter ermöglicht beispielsweise eine leichtere Befüllung des Reservoirs mit dem Stoff, insbesondere bei der Herstellung der erfindungsgemäßen Vorrichtung. Hierzu kann der Stoff bevorzugt mithilfe von Druckluft oder Vakuum in das Reservoir eingebracht werden, wobei der gasdurchlässige Filter den Durchtritt des Gases ermöglicht, aber den Stoff im Reservoir zurückhält.

Der Filter kann beispielsweise die Form einer Scheibe aufweisen. Der Filter weist bevorzugt eine offene Porosität auf. In einigen Ausführungsformen weist der Filter offene Poren mit einer Porengröße kleiner 5 µm, bevorzugt kleiner 3 µm und besonders bevorzugt kleiner 1 µm auf. Diese Porengröße kann durch den Ausschluss von sphärischen Partikeln mit einem entsprechenden Teilchendurchmesser bestimmt werden. Es handelt sich daher um eine nominelle Porengröße.

Der Filter kann durch eine Halterung innerhalb eines Reservoirs, zum Beispiel dem ersten und/oder zweiten Reservoir der Vorrichtung, befestigt sein. Die Halterung kann beispielsweise in Form eines Klemmrings ausgebildet sein. Gegebenenfalls kann der Filter in einer Aussparung in einer Wand des Reservoirs befestigt sein.

Der Filter kann beispielsweise Polyethylen, Polyurethan, Polyimid, Polyethersulfon, Polyvinylidenfluorid oder Poltetrafluorethylen aufweisen.

Der Filter kann ein Gewebefilter (woven filter) oder ein Vliesfilter (non-woven filter) sein.

In einigen Ausführungsformen umfasst das Reservoir einen zweiten Filter, der einen Innenraum der Vorrichtung in zwei voneinander getrennte Bereiche, das erste Reservoir und das zweite Reservoir, unterteilt.

Die Vorrichtung kann zwei Exemplare der oben beschriebenen Filter aufweisen, zum Beispiel einen Anschlussfilter und einen Zwischenfilter.

Ein Anschlussfilter kann an einem Ausgang des ersten Reservoirs angeordnet sein. Der Anschlussfilter kann eingerichtet sein, einen festen Stoff, zum Beispiel ein antiseptisches Oxidationsmittel und/oder ein Tensid, in der Vorrichtung zurückzuhalten, solange der Stoff nicht in einer Flüssigkeit gelöst ist.

Ein Zwischenfilter kann zwischen dem ersten Reservoir und dem zweiten Reservoir angeordnet sein, und das erste Reservoir fluidleitend mit dem zweiten Reservoir verbinden. Der Zwischenfilter kann dort durch eine Halterung befestigt sein. Das erste Reservoir kann ein antiseptisches Oxidationsmittel enthalten. Das zweite Reservoir kann ein Tensid enthalten.

In einer Ausführungsform enthält das erste Reservoir ein antiseptisches Oxidationsmittel, und das zweite Reservoir enthält ein Tensid. In einer Ausführungsform enthält das erste Reservoir ein Tensid, und das zweite Reservoir enthält ein antiseptisches Oxidationsmittel. Das antiseptische Oxidationsmittel und das Tensid befinden sich bevorzugt jeweils in fester Form, beispielsweise Pulverform. Eine solche Ausführungsform ermöglicht die Herstellung einer Wirkstofflösung mit einem antiseptischen Oxidationsmittel und einem Tensid, ohne dass die beiden Stoffe in fester Form während der Lagerung der Vorrichtung miteinander in Berührung kommen.

Das Reservoir kann derart dimensioniert sein, dass das Verhältnis der Volumina aus dem Gesamtvolumen des ersten und zweiten Reservoir, und des Behälters (d. h. [V(erstes Reservoir) + V(zweites Reservoir)] : V(Behälter)) in einem Verhältnis von 1:25 zu 1:500 liegt. Das Volumen des Behälters kann beispielsweise 250 ml, 500 ml oder 1000 ml betragen. Dementsprechend kann das Gesamtvolumen des ersten und zweiten Reservoirs beispielsweise 0,5 bis 10 ml, 1 bis 20 ml, oder 2 bis 40 ml betragen.

Die Vorrichtung kann weiterhin ein Fluidikelement aufweisen. Das Fluidikelement ist bevorzugt zur Aufnahme einer Flüssigkeit aus einem Behälter in das erste und/oder zweite Reservoir eingerichtet. Das Fluidikelement kann alternativ oder zusätzlich zur Abgabe einer Flüssigkeit aus dem Reservoir eingerichtet sein. Das Fluidikelement ist bevorzugt zum aktiven Fördern einer Flüssigkeit eingerichtet. Das Fluidikelement kann beispielsweise einen Kolben zum Fördern einer Flüssigkeit aufweisen. Das Fluidikelement kann eine Spritze oder eine Pumpe aufweisen.

In einer Ausführungsform weist das Fluidikelement einen innerhalb der Vorrichtung, beispielsweise innerhalb des ersten Reservoirs und/oder innerhalb des zweiten Reservoirs, beweglich angeordneten Kolben auf. Der Kolben schließt bevorzugt die Vorrichtung, beispielsweise das erste Reservoir und/oder das zweite Reservoir, flüssigkeitsdicht ab. Das Fluidikelement kann somit beispielsweise als Spritze oder Spritzenpumpe ausgeführt sein. Das Fluidikelement kann auch andere Arten von Mitteln zur Förderung einer Flüssigkeit aufweisen, z.B. eine Membranpumpe, Peristaltikpumpe, Druckluftkartusche, oder ein Anschluss für eine externe Druck- oder Vakuumquelle.

In einer Ausführungsform weist die Vorrichtung ein Adapterteil und ein Mischerteil auf, welche bevorzugt lösbar miteinander verbindbar sind. Die hierin beschriebenen Bestandteile der Vorrichtung können innerhalb eines solchen Adapterteils oder Mischerteils angeordnet sein. Beispielsweise kann das Adapterteil das Anschlusselement und den Dorn enthalten, und/oder das Mischerteil kann das Reservoir, den Filter und das Fluidikelement enthalten. Das Adapterteil und das Mischerteil können durch ein Verbindungselement, bevorzugt ein formschlüssiges Verbindungselement, beispielsweise mithilfe eines Gewindes oder Schnapphakens, miteinander verbindbar sein. Bevorzugt ermöglicht das Verbindungselement eine lösbare Verbindung des Adapterteil mit dem Mischerteil. Ein bevorzugtes Verbindungselement ist eine Luer-Lock-Verbindung. Eine Luer-Lock-Verbindung ermöglicht eine geschraubte Verbindung zweier jeweils kegelförmiger Verbindungsteile, die klemmfest ineinandergreifen. Eine Luer-Lock-Verbindung ist in US1742497A offenbart. Das Adapterteil kann bevorzugt ein Außengewinde aufweisen, das mit einem Innengewinde des Mischerteils verbindbar ist. Das Mischerteil kann ein Außengewinde aufweisen, das mit einem Innengewinde des Adapterteils verbindbar ist.

Das Adapterteil kann in einer Ausführungsform dazu eingerichtet sein, fluidleitend mit einem Behälter verbunden zu sein, während das Mischerteil vom Adapterteil gelöst und das Adapterteil gegebenenfalls mit einer anderen Vorrichtung verbunden wird, beispielsweise mit einem Verschluss oder einer Düse. Das Adapterteil kann somit als eine Art multifunktionaler Schnellverbinder für andere Komponenten dienen. Das Mischerteil kann eine Spritze umfassen, z.B. eine Spritze mit Luer-Lock-Verbindung. Das Mischerteil kann insbesondere eingerichtet sein, das antiseptische Oxidationsmittel und das Tensid mit einer Flüssigkeit zu mischen.

In einigen Ausführungsformen weist die Vorrichtung weiterhin ein Abgabeelement auf. Das Abgabeelement kann in dem Adapterteil angeordnet sein. Das Abgabeelement
kann eingerichtet sein, eine aus dem antiseptischen Oxidationsmittel und dem Tensid hergestellte desinfizierende Wundspüllösung aus der Vorrichtung abzugeben. Das Abgabeelement kann eingerichtet sein, die hergestellte Wundspüllösung in einem Zustand der Vorrichtung abzugeben, in welchem die Vorrichtung mit einem Behälter verbunden ist, wobei die Vorrichtung bevorzugt ein Adapterteil aufweist, das mit einem Mischerteil verbunden ist. Das Abgabeelement kann eine Durchführung aufweisen. Die Durchführung kann in dem Adapterteil parallel zu dem Dorn angeordnet sein.

Wie oben dargestellt kann die Vorrichtung ein Verbindungselement umfassen, um entweder einzelne Teile der erfindungsgemäßen Vorrichtung, insbesondere das Adapterteil und das Mischerteil, miteinander zu verbinden, oder die Vorrichtung mit anderen Vorrichtungen zu verbinden, oder beides. Hierbei kann entweder ein einziges Verbindungselement dazu eingerichtet sein, alternativ mit einer Vielzahl unterschiedlicher Vorrichtungen verbunden zu werden, oder es können mehrere Verbindungselemente an unterschiedlichen Positionen der Vorrichtung vorgesehen sein. Bevorzugt ist ein Verbindungselement ein formschlüssiges Verbindungselement. Ein Beispiel für ein solches Verbindungselement ist ein Gewinde. In einigen Ausführungsformen weist das Verbindungselement eine Luer-Lock-Verbindung auf.

In einer Ausführungsform weist die Vorrichtung ein Verbindungselement auf, mit dessen Hilfe das Adapterteil mit dem Mischerteil verbindbar ist. In einigen Ausführungsformen ist dasselbe Verbindungselement zusätzlich eingerichtet, die Vorrichtung mit einem Verschluss, einer Düse oder einer sonstigen Abgabevorrichtung zu verbinden. Alternativ können jeweils einzelne, voneinander verschiedene Verbindungselemente für diese Zwecke vorgesehen sein.

In einer Ausführungsform ist die Vorrichtung vollständig sterilisiert. In einer Ausführungsform ist die Vorrichtung vollständig pyrogenfrei. Der Begriff "sterilisiert" bezieht sich darauf, dass die Vorrichtung einem Verfahren unterzogen worden ist, um Krankheitserreger zu entfernen oder abzutöten, sodass die Vorrichtung gemäß der üblichen Standards wie zum Beispiel der Norm DIN EN 556-1 medizinisch verwendet werden kann. Verfahren zur Sterilisation umfassen beispielsweise Autoklavierung, Bestrahlung mit Elektronenstrahlen oder Gammastrahlen, oder Behandlung mit desinfizierenden Substanzen wie zum Beispiel Ethylenoxid. In ähnlicher Weise bezieht sich der Begriff "pyrogenfrei" darauf, dass die Vorrichtung einer im Fachgebiet üblichen Behandlung unterzogen worden ist, um Pyrogene zu entfernen oder zu zersetzen. Ob die Vorrichtung pyrogenfrei ist, kann mithilfe des Limulus-Amöbozytenlysat (LAL)-Tests gemäß Ph. Eur. Kapitel 2.6.14 geprüft werden. Hierin gelten jeweils die Normen bzw. Dokumente, die zum Prioritätstag der vorliegenden Anmeldung aktuell sind.

In einer Ausführungsform weist die Vorrichtung weiterhin eine Düse auf, welche zur Abgabe des Wirkstoffs in gelöster Form eingerichtet ist. Die Düse kann einen im Wesentlichen zylindrischen, an einem Ende spitz zulaufenden Hohlkörper aufweisen. Die Düse kann gegebenenfalls ein Gelenk umfassen, das durch seitliches Biegen einer Öffnung der Düse relativ zur gesamten Vorrichtung die Abgabe einer Flüssigkeit in unterschiedliche Richtungen erlaubt.

Bevorzugt ist die Düse lösbar fluidleitend mit dem Adapterteil verbindbar. Dies kann über ein formschlüssiges Verbindungselement, beispielsweise ein Gewinde, bevorzugt eine Luer-Lock-Verbindung, ermöglicht sein.

Die Vorrichtung kann weiterhin einen Schild umfassen. Der Schild kann eingerichtet sein, Teile der Vorrichtung vor Spritzern der abgegebenen Flüssigkeit, insbesondere einer Wirkstofflösung, zu schützen. Der Schild kann an oder in räumlicher Nähe zu der Düse angeordnet sein. Der Schild kann die Form einer Scheibe aufweisen. Der Schild kann eine konkave Krümmung aufweisen, ähnlich einer Parabolantenne. Der Schild kann eine zentral angeordnete Ausnehmung aufweisen, die zur Anbringung des Schilds an einem Ausgang der Vorrichtung eingerichtet ist.

Die Vorrichtung enthält bevorzugt ein antiseptisches Oxidationsmittel. In einer Ausführungsform ist das antiseptische Oxidationsmittel ausgewählt aus der Gruppe bestehend aus Chlorit, Hypochlorit und einem Peroxid. Das Chlorit ist bevorzugt ein Chloritsalz eines Alkalimetalls. Das Peroxid ist bevorzugt ein Peroxidsalz eines Alkalimetalls oder Erdalkalimetalls. Das Hypochlorit ist bevorzugt ein Hypochloritsalz eines Erdalkalimetalls. In einer Ausführungsform ist das antiseptische Oxidationsmittel ausgewählt aus der Gruppe bestehend aus Calciumhypochlorit (CAS 7778-54-3), Magnesiumhypochlorit (CAS 7778-54-3), Natriumchlorit (CAS 7758-19-2), Natriumhypochlorit (CAS 7681-52-9), Natriumperoxid (CAS 1313-60-6) und Calciumperoxid (CAS 1305-79-9). Die genannten Hypochlorit-Salze lösen sich in Wasser unter Freisetzung von Hypochlorit-Ionen, wobei die wässrigen Lösungen dieser Salze stark alkalisch sind. Durch Einwirkung von Säuren kann der pH-Wert dieser Lösungen auf ein physiologisch akzeptablen pH-Bereich von pH 4 bis pH 8 gesenkt werden. Hierzu kann die Vorrichtung bevorzugt einen pH-Regulator, insbesondere eine Säure, aufweisen. Im Neutralen und im sauren Milieu stehen die Hypochlorit-Ionen im Gleichgewicht mit Hypochloriger Säure. Natriumperoxid und auch Calciumperoxid lösen sich in Wasser ebenfalls und Bildung alkalischer Lösungen, in denen gelöste Peroxid-Anionen vorliegen. Durch Senkung des pH-Wertes infolge der Zugabe von Säuren stehen im neutralen und sauren Milieu die Peroxid-Anionen im Gleichgewicht mit Hydroperoxid-Ionen und Wasserstoffperoxid.

In einigen Ausführungsformen umfasst das antiseptische Oxidationsmittel Natriumchlorit und ein Hypochlorit-Salz. In einigen Ausführungsformen umfasst das antiseptische Oxidationsmittel Natriumchlorit und ein Peroxid. In einigen Ausführungsformen umfasst das antiseptische Oxidationsmittel Natriumchlorit, ein Hypochlorit-Salz und ein Peroxid. Die Vorrichtung kann bevorzugt eine Säure umfassen. Natriumchlorit (NaClO₂) bildet bei Einwirkung von Säuren Chlorige Säure, die mit Chlordioxid im Gleichgewicht steht. Chlorige Säure und auch Chlordioxid sind stark antiseptisch wirkende Oxidationsmittel.

In einer Ausführungsform liegt das antiseptische Oxidationsmittel und/oder das Tensid als Pulver mit einer mittleren Partikelgröße kleiner 300 µm, bevorzugt kleiner 200 µm und besonders bevorzugt kleiner 100 µm vor. Durch die kleine Partikelgröße kann eine schnelle Auflösung des betreffenden Stoffs erzielt werden. Die Partikelgröße des betreffenden Stoffs kann bevorzugt oberhalb des Ausschlussvolumens des Filters liegen. Dieser mittlere Durchmesser der Partikel kann durch fraktionierte Siebung bestimmt werden, wie es im Fachgebiet üblich ist. Die Partikelgrößenverteilung ist bevorzugt ausreichend schmal, so dass möglichst keine, z.B. weniger als 1% der Partikelmasse, durch den Filter gelangen kann. In einer Ausführungsform liegt der betreffende Stoff als zylinderförmiger Pressling oder als zylinderförmige erstarrte Schmelze vor.

In einer Ausführungsform weist das Anschlusselement eine erste Durchführung auf, welche zur Aufnahme einer Flüssigkeit aus einem Behälter in das Reservoir eingerichtet ist. Die erste Durchführung bildet bevorzugt eine fluidleitende Verbindung zwischen dem Anschlusselement, insbesondere dem Dorn, und dem Reservoir.

In einer Ausführungsform ist die Vorrichtung zur Abgabe einer Flüssigkeit aus einem Behälter eingerichtet. Hierbei ist bevorzugt die Abgabe in einem Zustand möglich, in welchem die Vorrichtung über die oben beschriebene erste Durchführung mit dem Reservoir fluidleitend verbunden ist. Beispielsweise kann mithilfe einer solchen Ausführungsform Flüssigkeit aus einem mit der Vorrichtung verbundenen Behälter zunächst in das Reservoir der Vorrichtung aufgenommen werden, der enthaltene Wirkstoff in der Flüssigkeit gelöst werden, und der gelöste Wirkstoff nachfolgend in den Behälter abgegeben werden, während die Vorrichtung mit dem Behälter verbunden ist. Anschließend kann der gelöste Wirkstoff über die Vorrichtung aus dem Behälter an einen Patienten abgegeben werden, während die Vorrichtung, insbesondere das Adapterteil der Vorrichtung, weiterhin mit dem Behälter verbunden ist. Gegebenenfalls kann die Vorrichtung zur Abgabe des gelösten Wirkstoffs mit einer Düse oder einer anderen Abgabevorrichtung, z.B. einem Jet-Lavage-System, verbunden werden, wobei ggf. das Mischerteil der Vorrichtung gegen die Düse oder andere Abgabevorrichtung ausgewechselt werden kann. Beispiele für Abgabesysteme sind beschrieben in EP2619415A1, EP2662146A2, Die Flüssigkeit ist bevorzugt eine medizinisch verträgliche Flüssigkeit. Die Flüssigkeit ist bevorzugt zur inneren Anwendung geeignet. In den Fällen, in denen die Vorrichtung mehrteilig ausgebildet ist, muss zur Abgabe einer Flüssigkeit aus einem Behälter nicht die gesamte Vorrichtung mit dem Behälter verbunden sein. Beispielsweise kann es genügen, wenn zur Abgabe der Flüssigkeit ein hierin beschriebener Adapterteil mit einem Behälter verbunden ist.

Ein Mischerteil kann beispielsweise nur zeitweise mit der Vorrichtung verbunden sein, um Flüssigkeit aus dem Behälter aufzunehmen und daraus eine Wundspüllösung herzustellen.

In einer Ausführungsform ist das erste Reservoir und/oder das zweite Reservoir durchlässig für Gammastrahlen und/oder Elektronenstrahlen. Hierdurch wird es ermöglicht, den Innenraum und die enthaltenen Stoffe der Vorrichtung, insbesondere antiseptische Oxidationsmittel und Tenside, mithilfe von Gammastrahlen und/oder Elektronenstrahlen zu sterilisieren, um eine vollständig sterilisierte Vorrichtung zu erhalten.

In einer Ausführungsform definieren der Dorn und das erste Reservoir gemeinsam eine Transferachse, wobei entlang dieser Transferachse eine Abgabe der Flüssigkeit aus dem Reservoir und/oder in das Reservoir erfolgen kann. In einer Ausführungsform definieren der Behälter und das erste Reservoir gemeinsam eine Transferachse, wobei entlang dieser Transferachse eine Abgabe der Flüssigkeit aus dem ersten Reservoir und/oder eine Aufnahme in das erste Reservoir erfolgen kann. Alternativ oder zusätzlich kann eine solche Transferachse in jeweils entsprechender Weise durch den Dorn und das zweite Reservoir definiert sein.

Ein weiterer Aspekt betrifft eine Vorrichtung zur Herstellung einer Wirkstofflösung, aufweisend
- ein Anschlusselement, das zum flüssigkeitsdichten Anschluss an einen Behälter ausgebildet und eingerichtet ist;
- einen Dorn, der zum Durchstechen eines Septums eines Behälters ausgebildet und eingerichtet ist;
- ein erstes Reservoir, das ein antiseptisches Oxidationsmittel in fester Form enthält, und über einen Ausgang des Reservoirs fluidleitend mit dem Anschlusselement verbindbar ist;
- einen flüssigkeitsdurchlässigen Anschlussfilter, der an dem Ausgang des ersten Reservoirs angeordnet ist, und eingerichtet ist, das antiseptische Oxidationsmittel in fester Form in dem Reservoir zurückzuhalten;
- ein zweites Reservoir, das ein Tensid in fester Form enthält;
- einen Zwischenfilter, der das erste Reservoir fluidleitend mit dem zweiten Reservoir verbindet;
- ein Fluidikelement, welches zur Aufnahme einer Flüssigkeit aus einem Behälter in das erste und/oder zweite Reservoir eingerichtet ist, und/oder zur Abgabe einer Flüssigkeit aus dem Reservoir eingerichtet ist;
wobei die Vorrichtung ausgebildet und eingerichtet ist, eine Flüssigkeit aus einem Behälter in das Reservoir aufzunehmen, den Wirkstoff in der Flüssigkeit zu lösen, und den gelösten Wirkstoff über das Anschlusselement an einen Patienten abzugeben.

Ein weiterer Aspekt betrifft ein Kit, aufweisend eine Vorrichtung gemäß einer der vorangehenden Ausführungsformen und einen Behälter mit einer medizinisch verträglichen Flüssigkeit, wobei die Vorrichtung lösbar mit dem Behälter verbindbar ist. Die Vorrichtung kann ein hierin beschriebenes Adapterteil und ein hierin beschriebenes Mischerteil umfassen. Das Adapterteil kann lösbar mit dem Mischerteil verbindbar sein. Das Adapterteil kann lösbar mit dem Behälter verbindbar sein. In einer Ausführungsform sind die Bestandteile des Kits, nämlich die Vorrichtung und der Behälter, gemeinsam keimdicht verpackt. In einer Ausführungsform sind die Bestandteile des Kits jeweils vollständig sterilisiert. In einer Ausführungsform sind die Bestandteile des Kits jeweils vollständig pyrogenfrei. Das Kit kann weiterhin eine Abgabevorrichtung zur Abgabe einer Flüssigkeit enthalten, beispielsweise eine Düse oder Spülvorrichtung, beispielsweise eine Spülvorrichtung gemäß EP4035605A1. Die medizinisch verträgliche Flüssigkeit kann eine Infusionslösung oder medizinische Spüllösung umfassen. Die medizinisch verträgliche Flüssigkeit kann eine physiologische Kochsalzlösung, eine Ringer-Lösung, oder eine vergleichbare kochsalzhaltige Lösung umfassen. Die medizinisch verträgliche Flüssigkeit ist bevorzugt steril und/oder pyrogenfrei.

Ein weiterer Aspekt betrifft einen Wirkstoff zur Verwendung in einem medizinischen Behandlungsverfahren, wobei der Wirkstoff mithilfe einer hierin beschriebenen Vorrichtung unmittelbar vor der Verabreichung an einen Patienten in einer Flüssigkeit gelöst und anschließend in gelöster Form verabreicht wird. Der Wirkstoff kann ein Stoff sein, welcher eine pharmakologische Wirkung aufweist. Der Wirkstoff kann beispielsweise eine antimikrobielle Wirkung aufweisen. Der Wirkstoff kann ein antiseptisches Oxidationsmittel und ggf. weiterhin ein Tensid umfassen. In einer Ausführungsform ist das antiseptische Oxidationsmittel ausgewählt aus der Gruppe bestehend aus Chlorit, Hypochlorit und einem Peroxid. Das Chlorit ist bevorzugt ein Chloritsalz eines Alkalimetalls. Das Peroxid ist bevorzugt ein Peroxidsalz eines Alkalimetalls oder Erdalkalimetalls. Das Hypochlorit ist bevorzugt ein Hypochloritsalz eines Erdalkalimetalls. In einer Ausführungsform ist das antiseptische Oxidationsmittel ausgewählt aus der Gruppe bestehend aus Calciumhypochlorit (CAS 7778-54-3), Magnesiumhypochlorit (CAS 7778-54-3), Natriumchlorit (CAS 7758-19-2), Natriumhypochlorit (CAS 7681-52-9), Natriumperoxid (CAS 1313-60-6) und Calciumperoxid (CAS 1305-79-9). Der Wirkstoff kann mithilfe einer hierin beschriebenen Vorrichtung verabreicht werden. Dazu kann beispielsweise ein hierin verwendetes Adapterteil mit einer Düse oder einem Jet-Lavagesystem verbunden werden. Das Behandlungsverfahren kann beispielsweise die Behandlung einer Infektion einer chirurgischen Wunde sein, wie nachfolgend ausführlicher dargelegt ist.

Ein weiterer Aspekt betrifft ein medizinisches Behandlungsverfahren, wobei ein Wirkstoff, insbesondere ein antiseptisches Oxidationsmittel und weiterhin ein Tensid, mithilfe einer hierin beschriebenen Vorrichtung unmittelbar vor der Verabreichung an einen Patienten wie hierin beschrieben in einer Flüssigkeit gelöst und anschließend in gelöster Form verabreicht wird. Die Flüssigkeit ist bevorzugt eine medizinisch verträgliche Flüssigkeit wie hierin beschrieben. Das Behandlungsverfahren kann beispielsweise die Desinfektion eines Gewebes umfassen. Das Behandlungsverfahren kann eine chirurgische Gelenkoperation umfassen, beispielsweise die Implantation oder der Austausch eines Gelenkimplantats. Das Gelenkimplantat kann beispielsweise ein Spacer sein. Die Gelenkoperation kann beispielsweise eine chirurgische Hüft-, Knie-, Schulter- oder Rückenoperation umfassen. Das Behandlungsverfahren kann ferner die Therapie eines entzündlichen Zustands umfassen. Das Behandlungsverfahren kann beispielsweise die Behandlung einer Gelenkarthrose oder einer Gelenkinfektion umfassen.

Ein weiterer Aspekt betrifft eine Zusammensetzung zur Anwendung in einem Verfahren zur Behandlung einer chirurgischen Wunde, insbesondere einer infizierten Wunde, wobei das Verfahren die Verabreichung der Zusammensetzung mit einer hierin beschriebenen Vorrichtung umfasst. Die Zusammensetzung umfasst bevorzugt ein antiseptisches Oxidationsmittel und ein Tensid. Die Behandlung kann eine chirurgische Gelenkoperation umfassen, beispielsweise die Implantation oder der Austausch eines Gelenkimplantats. Das Gelenkimplantat kann beispielsweise ein Spacer sein. Die Gelenkoperation kann beispielsweise eine chirurgische Hüft-, Knie-, Schulter- oder Rückenoperation umfassen. Die Behandlung kann ferner die Therapie eines entzündlichen Zustands umfassen. Die Behandlung kann beispielsweise die Behandlung einer Gelenkarthrose oder einer Gelenkinfektion umfassen.

Ein weiterer Aspekt betrifft ein Verfahren zur Herstellung einer Wirkstofflösung, welches die nachfolgenden Schritte umfasst,
a) Aufnahme einer Flüssigkeit aus einem Behälter mithilfe einer hierin beschriebenen Vorrichtung in das erste Reservoir und das zweite Reservoir der Vorrichtung,
b) Mischen eines antiseptischen Oxidationsmittels und eines Tensids innerhalb der Vorrichtung mit der in Schritt a) aufgenommenen Flüssigkeit,
c) dadurch Erhalt einer Wirkstofflösung,
d) gegebenenfalls Abgabe der Wirkstofflösung aus der Vorrichtung in den Behälter.

Das Verfahren kann bevorzugt in der Reihenfolge a), b), c) oder a), b), c), d) durchgeführt werden. Das Verfahren kann weiterhin einen Schritt umfassen, in welchem die Wirkstofflösung aus der Vorrichtung oder aus dem Behälter abgegeben wird. Bevorzugt kann die Wirkstofflösung aus dem Behälter über die Vorrichtung abgegeben werden, wie hierin andernorts ausführlicher beschrieben. Die Wirkstofflösung kann eine Wundspüllösung sein. Die Lösung kann weiterhin einen pH-Regulator umfassen. Das antiseptisches Oxidationsmittel kann ein Chlorit, ein Hypochlorit und/oder ein Peroxid umfassen. Das Tensid kann ein quartäres Ammoniumsalz und/oder ein Fettalkoholsulfat umfassen.

Ein weiterer Aspekt betrifft eine Wirkstofflösung, die nach einem oben dargestellten Verfahren herstellbar ist. Die Wirkstofflösung kann ein antiseptisches Oxidationsmittel und ein Tensid umfassen. Die Wirkstofflösung kann eine Wundspüllösung sein. Die Lösung kann weiterhin einen pH-Regulator umfassen. Das antiseptisches Oxidationsmittel kann ein Chlorit, ein Hypochlorit und/oder ein Peroxid umfassen. Das Tensid kann ein quartäres Ammoniumsalz und/oder ein Fettalkoholsulfat umfassen.

Ein weiterer Aspekt betrifft eine Verwendung einer hierin beschriebenen Vorrichtung oder eines hierin beschriebenen Kits zur Herstellung einer Wirkstofflösung, insbesondere einer antiseptischen Wundspüllösung. Die Wirkstofflösung kann ein antiseptisches Oxidationsmittel und ein Tensid umfassen. Die Wirkstofflösung kann eine Wundspüllösung sein. Die Lösung kann weiterhin einen pH-Regulator umfassen. Das antiseptisches Oxidationsmittel kann ein Chlorit, ein Hypochlorit und/oder ein Peroxid umfassen. Das Tensid kann ein quartäres Ammoniumsalz und/oder ein Fettalkoholsulfat umfassen.

Ein weiterer Aspekt betrifft eine medizinische Wundspüllösung zur Anwendung in einem Verfahren für die Prophylaxe oder Behandlung einer Wundinfektion, wobei die medizinische Wundspüllösung ein antiseptisches Oxidationsmittel und ein Tensid umfasst. Die Wundinfektion kann eine Infektion einer inneren Wunde sein, beispielsweise einer Operationswunde. Die Wundinfektion kann die Infektion eines Knochengewebes umfassen. Ein Knochengewebe kann insbesondere Knochen, Gelenke und Knorpel umfassen. Die Lösung kann weiterhin einen pH-Regulator umfassen. Das antiseptisches Oxidationsmittel kann ein Chlorit, ein Hypochlorit und/oder ein Peroxid umfassen. Das Tensid kann ein quartäres Ammoniumsalz und/oder ein Fettalkoholsulfat umfassen.

Ein weiterer Aspekt betrifft eine medizinische Wundspüllösung zur Anwendung als desinfizierendes Agens, wobei die medizinische Wundspüllösung ein antiseptisches Oxidationsmittel und ein Tensid umfasst. Die Lösung kann weiterhin einen pH-Regulator umfassen. Das antiseptisches Oxidationsmittel kann ein Chlorit, ein Hypochlorit und/oder ein Peroxid umfassen. Das Tensid kann ein quartäres Ammoniumsalz und/oder ein Fettalkoholsulfat umfassen.

### BEISPIELE

Die Erfindung wird nachfolgend anhand von Beispielen weiter verdeutlicht, die jedoch nicht als einschränkend zu verstehen sind. Dem Fachmann wird ersichtlich sein, dass anstelle der hier beschriebenen Merkmale andere äquivalente Mittel in ähnlicher Weise verwendet werden können.

### FIGUREN

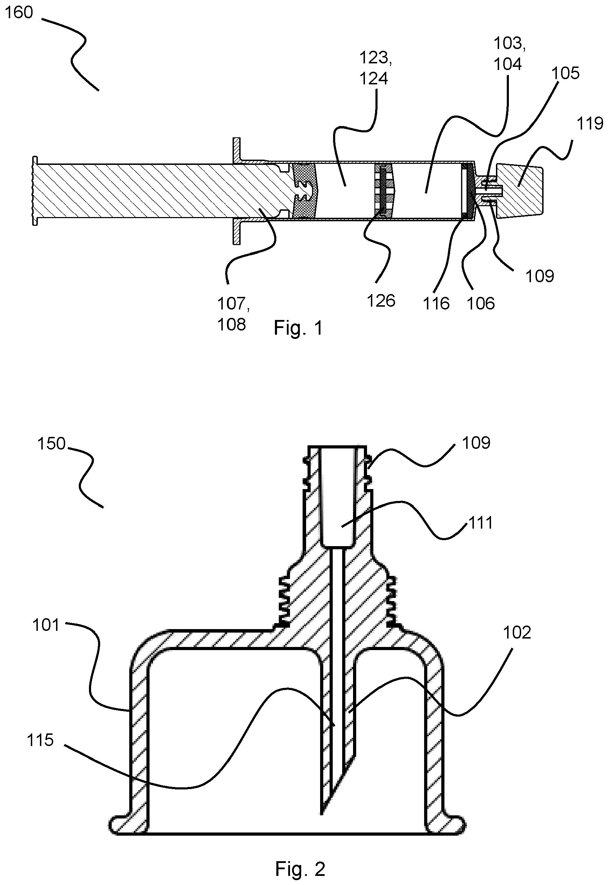

**Figur 1** zeigt ein Mischerteil **160** einer erfindungsgemäßen Vorrichtung mit einem ersten Reservoir **103,** einem zweiten Reservoir **123** und einem Fluidikelement **107** zum Transport einer Flüssigkeit **202** aus einem Behälter **200** (in dieser Figur nicht dargestellt). Das Fluidikelement **107** ist hier als Spritze ausgeführt, die einen Spritzenkolben **108** aufweist. Ein Wirkstoff wie z.B. ein antiseptisches Oxidationsmittel **104** ist als Pulver in dem ersten Reservoir **103** angeordnet. Die Vorrichtung umfasst weiterhin einen Anschlussfilter **106,** um den Wirkstoff **104** in dem ersten Reservoir **103** zu halten. Ein Ausgang **105** des ersten Reservoirs ist eingerichtet, eine fluidleitende Verbindung des ersten Reservoirs **103** mit einem Adapterteil der Vorrichtung herzustellen. Der Wirkstoff **104** kann den Anschlussfilter **106** nur in gelöster Form passieren. Der Anschlussfilter **106** ist durch eine Halterung **116** an dem ersten Reservoir **103** befestigt, um das Reservoir **103** nach außen abzudichten, sodass kein fester Wirkstoff **104** durch den Filter **106** aus dem ersten Reservoir **103** gelangen kann. Das Mischerteil **160** weist ein erstes Verbindungselement **109** zum Anschluss an ein Adapterteil **150** auf. Dieses Verbindungselement des Mischerteils **160** ist komplementär zu einem Verbindungselement des Adapterteils **150** ausgestaltet. Im hier gezeigten Beispiel weist das Mischerteil **160** ein Luer-Lock-Innengewinde auf. Das erste Verbindungselement **109** kann auch lösbar mit einem ersten Verschluss **119** verbindbar sein, um das erste Reservoir **103** und den Ausgang **105** flüssigkeitsdicht zu verschließen. Das erste Verbindungselement **109** weist hier ein Gewinde auf, um eine lösbare, flüssigkeitsdichte Verbindung herzustellen. Das Mischerteil **160** weist ein zweites Reservoir **123** auf, welches ein Tensid **124** in Pulverform enthält. Ein Zwischenfilter **126** ist zwischen dem ersten Reservoir **103** und dem zweiten Reservoir **123** angeordnet, und stellt eine fluidleitende Verbindung zwischen dem ersten Reservoir **103** und dem zweiten Reservoir **123** her. Der Zwischenfilter **126** ist durch eine Halterung an dem ersten Reservoir **103** befestigt, um das Reservoir **103** nach außen abzudichten, sodass kein fester Wirkstoff **104** durch den Filter **106** aus dem ersten Reservoir **103** gelangen kann. Das Fluidikelement **107** ist eingerichtet, Flüssigkeit in das erste Reservoir **103** und das zweite Reservoir **123** zu fördern, um den Wirkstoff **104** und das Tensid **124** in der Flüssigkeit zu lösen, um eine Wundspüllösung herzustellen. Das Fluidikelement **107** ist weiterhin eingerichtet, diese Wundspüllösung aus der Vorrichtung abzugeben.
**Figur 2** zeigt beispielhaft ein Adapterteil **150** einer Vorrichtung, welches mit einem Behälter **200** verbindbar ist. Das Adapterteil **150** der Vorrichtung weist ein Anschlusselement **101** auf, welches formschlüssig mit einem Behälter verbunden werden kann, beispielsweise mit einem dafür vorgesehenen Anschluss am Kopf einer Infusionsflasche oder eines Folienbeutels. Die Vorrichtung weist weiterhin einen Dorn **102** auf, mit dem ein Septum **201** eines Behälters durchstochen werden kann. Der Dorn **102** weist einen inneren Hohlraum **115** auf, um eine Flüssigkeit aus dem Behälter in die Vorrichtung zu leiten. Der innere Hohlraum **115** ist mit einer Durchführung **111** der Vorrichtung fluidleitend verbunden. Das Adapterteil **150** der Vorrichtung **100** weist weiterhin ein Verbindungselement **109** auf, mit dem das Adapterteil **150** der Vorrichtung mit einem Mischerteil **160** der Vorrichtung verbindbar ist. Das Verbindungselement **109** ist in diesem Beispiel als Luer-Lock-Außengewinde ausgeführt.

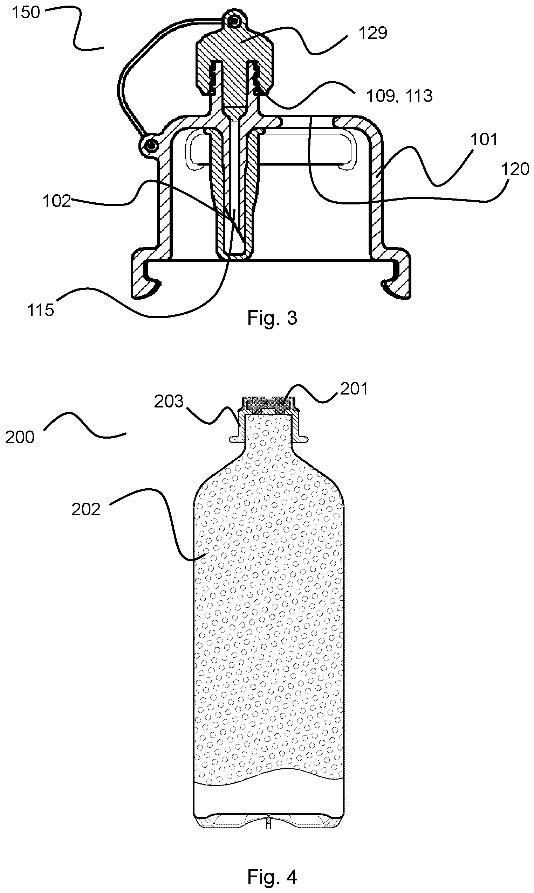

**Figur 3** zeigt beispielhaft ein Adapterteil **150,** welches für Verpackungs- und Transportzwecke eine Abdeckung aufweist, die den Dorn **102** umgibt und somit den Hohlraum **115** des Dorns an einer ersten Seite des Hohlraums flüssigkeitsdicht verschließt. In der hier gezeigten Ausführungsform weist das Adapterteil nur ein einziges Gewinde auf, das wahlweise mit einem Mischerteil, einem zweiten Verschluss **129,** einer Düse oder einer sonstigen Abgabevorrichtung wie z.B. einem Jet-Lavagesystem lösbar verbindbar ist. Somit kann das Gewinde gleichzeitig die Funktionen des ersten Verbindungselements **109** und des zweiten Verbindungselements **113** vereinen. Der Verschluss **129** verschließt den Hohlraum **115** des Dorns an einerzweiten Seite des Hohlraums flüssigkeitsdicht. Eine Abgabevorrichtung kann auch über das Abgabeelement **120** mit dem Adapterteil verbunden werden. Das Abgabeelement **120** weist hier eine Durchführung auf, die sich parallel zum Dorn **102** in Richtung des Behälters erstreckt. Beispielsweise kann eine Abgabevorrichtung, die einen ähnlichen hohlen Dorn aufweist, mit dem Abgabeelement **120** verbunden werden und das Septum des Behälters parallel zum Dorn **102** des Adapterteils durchstechen.
**Figur 4** zeigt einen Behälter **200,** der mit einer erfindungsgemäßen Vorrichtung verbunden werden kann. Der Behälter **200** enthält eine medizinisch verträgliche Flüssigkeit **202,** und weist ein Septum **201** auf, welches den Behälter nach außen abdichtet. Das Septum **201** ist aus einem elastischen Material, beispielsweise Silikon, gebildet. Das Septum **201** ist mithilfe des Dorns **102** der Vorrichtung durchstechbar. Das Septum kann zusätzlich durch ein Siegel geschützt sein, das vor der Verbindung mit der erfindungsgemäßen Vorrichtung entfernt werden kann. In der hier gezeigten Ausführungsform ist der Behälter eine Infusionsflasche aus Kunststoff. Es können aber auch andere Behälter wie z.B. Folienschlauchbeutel verwendet werden, in denen medizinische Lösungen wie z.B. eine sterile Kochsalzlösung, kommerziell erhältlich sind, und die mit einem Dorn **102** durchstechbar sind.

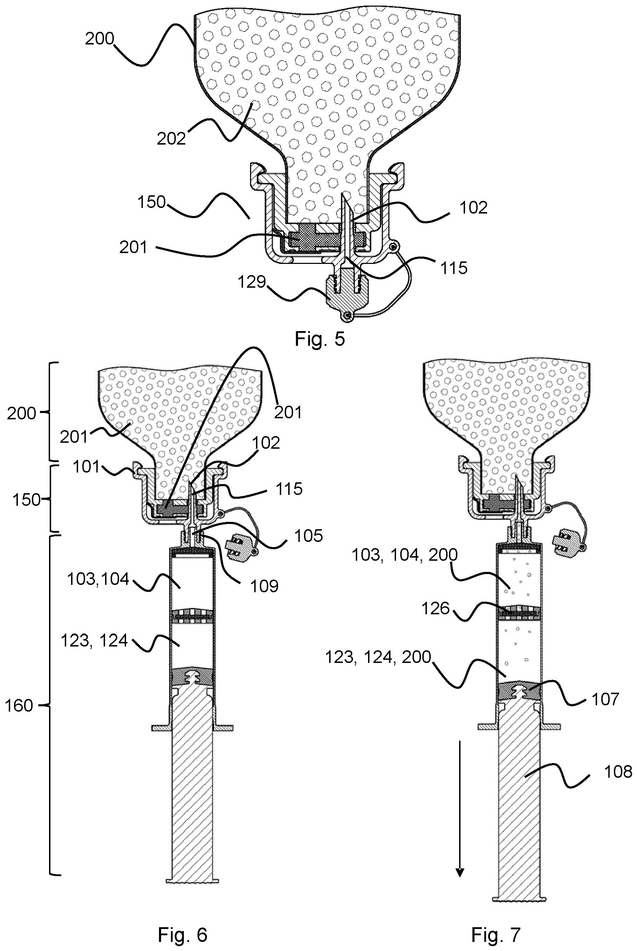

**Figur 5** zeigt ein Adapterteil **150** einer erfindungsgemäßen Vorrichtung, welche an einen Behälter **200** angebracht ist. Der Dorn **102** durchdringt das Septum **201** des Behälters **200,** sodass die Flüssigkeit **202** aus dem Behälter durch den Hohlraum **115** und die erste Durchführung **109** in die Vorrichtung überführt werden kann, insbesondere wenn das Adapterteil mit dem Mischerteil verbunden ist. Das Adapterteil **150** ist mithilfe eines Schnappverschlusses lösbar mit dem Behälter **200** verbunden. Der Schnappverschluss umgibt dabei den Kragen der Plastikflasche.
**Figur 6** zeigt eine erfindungsgemäße Vorrichtung **100,** die mit einem Behälter **200** verbunden ist. Das Adapterteil **150** der Vorrichtung ist über ein Verbindungselement **109,** das hier als Gewinde ausgeführt ist, mit dem Mischerteil **160** der Vorrichtung fluidleitend und flüssigkeitsdicht verbunden. Das Anschlusselement **101** ist mit dem Behälter **200** verbunden, wobei der Dorn **102** durch ein Septum **201** des Behälters in den Behälter **200** eingeführt ist. Der Innenraum des Behälters ist durch einen Hohlraum **115** des Dorns **102** mit dem Reservoir **103** fluidleitend verbunden. Figur 6 stellt somit auch einen ersten Schritt eines Verfahrens zur Herstellung einer Wundspüllösung dar.
**Figur 7** zeigt eine erfindungsgemäße Vorrichtung **100,** die mit einem Behälter **200** verbunden ist. Mithilfe eines Fluidikelement, das hier als Spritze ausgeführt ist, welche einen Kolben **108** aufweist, kann eine Flüssigkeit **201** aus dem Behälter **200** in das erste Reservoir **103** und das zweite Reservoir **203** gezogen werden, so dass sich ein antiseptisches Oxidationsmittel **103,** das im ersten Reservoir **103** angeordnet ist, und ein Tensid **124,** das im zweiten Reservoir **203** angeordnet ist, in der Flüssigkeit **201** lösen. Die Pfeilrichtung gibt hierbei die Bewegungsrichtung des Spritzenkobens an, wenn Flüssigkeit in den Mischerteil der Vorrichtung gezogen wird. Erst im gelösten Zustand können diese beiden Stoffe den Zwischenfilter **126** passieren, der zwischen dem ersten Reservoir **103** und dem zweiten Reservoir **203** angeordnet ist. Gemeinsam mit der Flüssigkeit bilden diese gelösten Stoffe eine Wundspüllösung, die ein antiseptisches Oxidationsmittel und ein Tensid umfasst. Figur 7 stellt somit auch einen zweiten Schritt eines Verfahrens zur Herstellung einer Wundspüllösung dar.

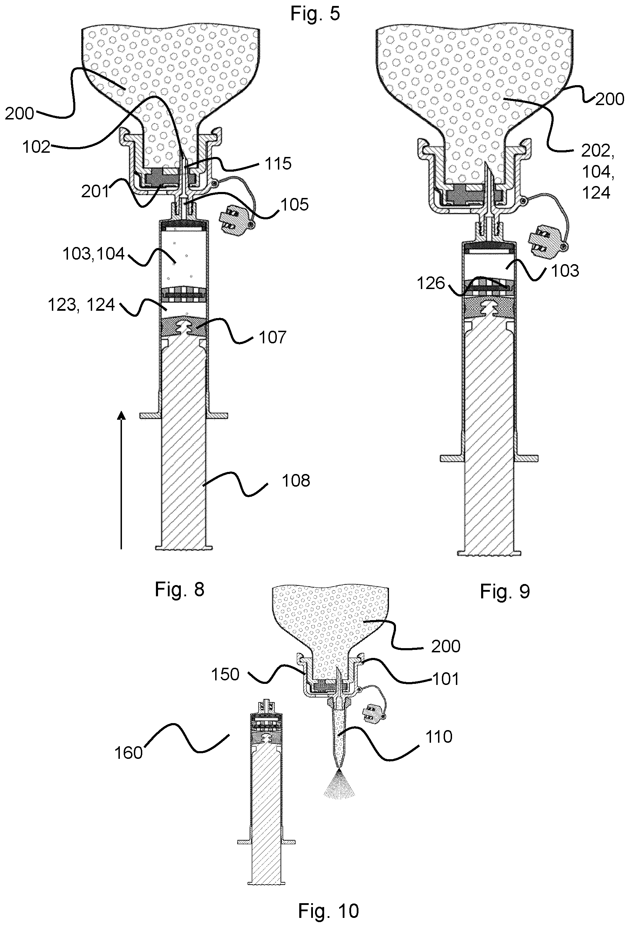

**Figur 8** zeigt eine erfindungsgemäße Vorrichtung **100,** die mit einem Behälter **200** verbunden ist. Der Spritzenkolben **107** wird hier in Pfeilrichtung bewegt, um eine in der Mischvorrichtung hergestellte Wundspüllösung aus dem ersten Reservoir **103** und dem zweiten Reservoir **123** des Mischerteils **160** über das Adapterteil **150** in den Behälter **200** abzugeben. Figur 8 stellt somit auch einen dritten, optionalen Schritt eines Verfahrens zur Herstellung einer Wundspüllösung dar, in welchem eine Wundspüllösung in einem Behälter erhalten wird.
**Figur 9** zeigt eine erfindungsgemäße Vorrichtung **100,** die mit einem Behälter **200** verbunden ist. In der hier gezeigten Ausführungsform ist der Zwischenfilter **126** beweglich in dem Mischerteil angeordnet, so dass das erste Reservoir **103** und das zweite Reservoir **123** vollständig entleert werden können, und die gesamte in der Vorrichtung hergestellte Wundspüllösung aus der Vorrichtung **100** in den Behälter **200** abgegeben werden kann. Hierzu wird der Zwischenfilter **126** mithilfe des Spritzenkolbens **107** in Richtung des Ausgangs **105** des ersten Reservoirs bewegt, bis der Zwischenfilter **126** mit dem Ausgang **105** abschließt. Figur 9 stellt somit auch einen vierten, optionalen Schritt eines Verfahrens zur Herstellung einer Wundspüllösung dar, in welchem eine Wundspüllösung in einem Behälter erhalten wird.
**Figur 10** zeigt ein Adapterteil **150,** die gleichzeitig mit einem Behälter **200** und mit einer Düse **110** verbunden ist. Hierzu umfasst das Adapterteil **150** eine erste Durchführung **111,** die zum Anschluss an eine Düse **110** oder eine andere externe Abgabevorrichtung eingerichtet ist. In der hier gezeigten Konfiguration ist das Adapterteil **150** mithilfe des Anschlusselements **101** mit einem Behälter **200** fluidleitend verbunden, sodass eine mithilfe der Vorrichtung hergestellte Wirkstofflösung aus dem Behälter **200** über die erste Durchführung **111** abgegeben werden kann.

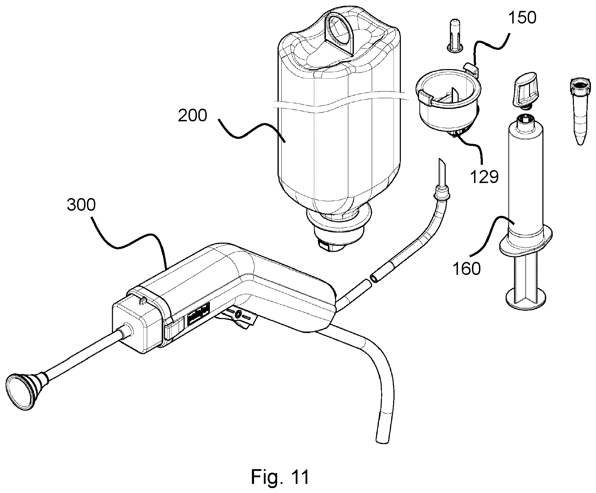

**Figur 11** zeigt ein Kit, welches neben einer erfindungsgemäßen Vorrichtung aus Adapterteil **150** und Mischerteil **160** weiterhin einen Behälter **200** mit einer medizinisch verträglichen Flüssigkeit, z.B. sterile Kochsalzlösung enthält. Weiterhin enthält das Kit eine Jet-Lavage-Vorrichtung **300,** mit deren Hilfe eine mit Hilfe der Vorrichtung hergestellte Wundspüllösung direkt an einen Patienten verabreicht werden kann, z.B. während eines chirurgischen Eingriffs. Die Jet-Lavage-Vorrichtung **300** kann dazu über einen zweiten Verschluss **129** des Adapterteils **150** mit dem Behälter **200** verbunden werden, nachdem wie in den Figuren 6 bis 9 gezeigt eine Wundspüllösung mithilfe der Vorrichtung hergestellt worden und in den Behälter **200** abgegeben ist. Alternativ kann die Wundspüllösung wie in Figur **10** gezeigt über eine Düse **110** abgegeben werden, die mit dem Adapterteil **150** verbindbar ist.

### AUSFÜHRUNGSBEISPIELE

Für die Ausführungsbeispiele wurden Calciumhypochlorit (CAS 7778-54-3; Hypochlorit-Gehalt 68 %), wasserfreie Citronensäure (CAS 77-92-9), wasserfreie Glucuronsäure, (576-37-4) Natriumchlorit (7758-19-2), Benzalkoniumchlorid (CAS 63449-41-2) und Natriumdodecylsulfat (CAS 151-21-3), jeweils bezogen von Sigma-Aldrich, sowie handelsübliche, sterile 500-ml-Plastikinfusionsflaschen mit jeweils 500 ml 0,9%iger Kochsalzlösung verwendet. Die Plastikinfusionsflaschen besaßen jeweils zwei Septen am Kopf der Flasche. Alle in den Tabellen angegebenen ppm-Werte sind Massenanteile (d.h. Masse des gelösten Stoffs pro Masse der Lösung). Das Calciumperoxid hatte eine Peroxid-Gehalt von 44 Prozent. Das Natriumchlorit hatte einen Chlorit-Gehalt von 67 %.

Aus einer handelsüblichen, medizinischen 20-ml-Kunststoffspritze wurde eine Mischvorrichtung konstruiert, indem der Innenraum der Spritze durch Einbau einer PE-Porenschreibe in ein erstes Reservoir und ein zweites Reservoir unterteilt wurde. Die jeweiligen Komponenten der Versuche 1-24 wurden, wie in den nachfolgenden Tabellen angegeben, jeweils in das erste bzw. zweite Reservoir eingewogen und danach die Spritze mit einem hierin beschriebenen Adapterteil gemäß Figur 2 verschraubt. Das Adapterteil wurde auf den Kopf der Plastikinfusionsflasche gedrückt, wobei der Stechdorn ein Septum der Plastikinfusionsflasche perforierte. Nach der Verbindung des Adapterteils mit der Plastikinfusionsflasche wurde diese in Richtung des Flaschenkopfs gekippt und mit dem Kolben der Spritze wurde fünfmal Flüssigkeit aus der Plastikinfusionsflasche aufgezogen und anschließend wieder zurück in die Plastikflasche gedrückt. Dabei lösten sich die Komponenten im ersten Reservoir und im zweiten Reservoir vollständig, so dass jeweils eine frisch hergestellte Wundspüllösung mit antiseptischem Oxidationsmittel, pH-Regulator und Tensid erhalten wurde. Die erhaltene Flüssigkeit in der Plastikinfusionsflasche war bei allen Beispielen sichtklar. Danach wurde die Spritze von dem Adapterteil abgeschraubt und eine Ausspritztülle auf das Adapterteil aufgeschraubt. Der pH-Wert der so hergestellten Lösungen lag zwischen pH 5 und pH 7. Die jeweils eingesetzten Konzentrationen von antiseptischem Oxidationsmittel, pH-Regulator und Tensid, sowie die Konzentrationen der entsprechenden Stoffe (ppm-Angaben hierbei jeweils als Anteil Masse/Masse) in der daraus hergestellten Wundspüllösung sind in den nachfolgenden Tabellen dargestellt.

**Tabelle 1: Eingesetzte Mengen von Calciumhypochlorit, Citronensäure und Natriumlaurylsulfat in den Beispielen 1 bis 13.**

| | **Reservoir 1** | **Reservoir 2** | |
|---|---|---|---|
| | **Calciumhypochlorit** | **Citronensäure** | **Natriumlaurylsulfat** |
| **Beispiel Nr.** | **[g]** | **[g]** | **[g]** |
| 1 | 0,074 | 0,056 | 0,050 |
| 2 | 0,147 | 0,110 | 0,050 |
| 3 | 0,294 | 0,220 | 0,050 |
| 4 | 0,735 | 0,551 | 0,050 |
| 5 | 1,471 | 1,103 | 0,050 |
| 6 | 2,941 | 2,206 | 0,050 |
| 7 | 0,074 | 0,056 | 0,100 |
| 8 | 0,147 | 0,110 | 0,100 |
| 9 | 0,294 | 0,220 | 0,100 |
| 10 | 0,735 | 0,551 | 0,100 |
| 11 | 1,471 | 1,103 | 0,100 |
| 12 | 2,941 | 2,206 | 0,100 |
| 13 | 1,471 | 1,103 | 0,200 |

**Tabelle 2: Konzentrationen von Hypochlorit, Citronensäure und Natriumlaurylsulfat in der hergestellten Lösung in den Beispielen 1 bis 13.**

| **Beispiel Nr.** | **Konzentrationen in der Lösung** | | |
|---|---|---|---|
| | **Hypochlorit** | **Citronensäure** | **Natriumlaurylsulfat** |
| | **[ppm]** | **[ppm]** | **[ppm]** |
| 1 | 100 | 112 | 100 |
| 2 | 200 | 220 | 100 |
| 3 | 400 | 440 | 100 |
| 4 | 1000 | 1102 | 100 |
| 5 | 2000 | 2206 | 100 |
| 6 | 4000 | 4412 | 100 |
| 7 | 100 | 112 | 200 |
| 8 | 200 | 220 | 200 |
| 9 | 400 | 440 | 200 |
| 10 | 1000 | 1102 | 200 |
| 11 | 2000 | 2206 | 200 |
| 12 | 4000 | 4412 | 200 |
| 13 | 2000 | 2206 | 400 |

**Tabelle 3: Eingesetzte Mengen von Calciumhypochlorit, Citronensäure und Benzalkoniumchlorid in den Beispielen 14 bis 16.**

| | **Reservoir 1** | **Reservoir 2** | |
|---|---|---|---|
| | **Calciumhypochlorit** | **Citronensäure** | **Benzalkoniumchlorid** |
| **Beispiel Nr.** | **[g]** | **[g]** | **[g]** |
| 14 | 0,074 | 0,052 | 0,050 |
| 15 | 0,147 | 0,103 | 0,050 |
| 16 | 0,294 | 0,206 | 0,050 |

**Tabelle 4: Konzentrationen von Hypochlorit, Citronensäure und Natriumlaurylsulfat in der hergestellten Lösung in den Beispielen 14 bis 16.**

| **Beispiel Nr.** | **Konzentrationen in der Lösung** | | |
|---|---|---|---|
| | **Hypochlorit** | **Citronensäure** | **Benzalkoniumchlorid** |
| | **[ppm]** | **[ppm]** | **[ppm]** |
| 14 | 100 | 104 | 100 |
| 15 | 200 | 206 | 100 |
| 16 | 400 | 412 | 100 |

**Tabelle 5: Eingesetzte Mengen von Calciumhypochlorit, Glucuronsäure und Natriumlaurylsulfat in den Beispielen 17 bis 19.**

| | **Reservoir 1** | **Reservoir 2** | |
|---|---|---|---|
| | **Calciumhypochlorit** | **Glucuronsäure** | **Natriumlaurylsulfat** |
| **Beispiel Nr.** | **[g]** | **[g]** | **[g]** |
| 17 | 0,074 | 0,052 | 0,050 |
| 18 | 0,147 | 0,103 | 0,050 |
| 19 | 0,294 | 0,206 | 0,050 |

**Tabelle 6: Konzentrationen von Hypochlorit, Glucuronsäure und Natriumlaurylsulfat in der hergestellten Lösung in den Beispielen 17 bis 19.**

| **Beispiel Nr.** | **Konzentrationen in der Lösung** | | |
|---|---|---|---|
| | **Hypochlorit** | **Glucuronsäure** | **Natriumlaurylsulfat** |
| | **[ppm] Hypochlorit** | **[ppm]** | **[ppm]** |
| 17 | 100 | 104 | 100 |
| 18 | 200 | 206 | 100 |
| 19 | 400 | 412 | 100 |

**Tabelle 7: Eingesetzte Mengen von Calciumperoxid, Citronensäure und Natriumlaurylsulfat in den Beispielen 20 bis 22.**

| **Beispiel Nr.** | **Reservoir 1** | **Reservoir 2** | |
|---|---|---|---|
| | **Calciumperoxid** | **Citronensäure** | **Natriumlaurylsulfat** |
| | **[g]** | **[g]** | **[g]** |
| 20 | 226 | 0,052 | 0,050 |
| 21 | 451 | 0,103 | 0,050 |
| 22 | 903 | 0,206 | 0,050 |

**Tabelle 8: Konzentrationen von Peroxid, Citronensäure und Natriumlaurylsulfat in der hergestellten Lösung in den Beispielen 20 bis 22.**

| | **Konzentrationen in der Lösung** | | |
|---|---|---|---|
| | **Peroxid** | **Citronensäure** | **Natriumlaurylsulfat** |
| **Beispiel Nr.** | **[ppm]** | **[ppm]** | **[ppm]** |
| 20 | 100 | 104 | 100 |
| 21 | 200 | 206 | 100 |
| 22 | 400 | 412 | 100 |

**Tabelle 9: Eingesetzte Mengen von Natriumchlorit, Citronensäure und Natriumlaurylsulfat in den Beispielen 23 und 24.**

| | **Reservoir 1** | **Reservoir 2** | |
|---|---|---|---|
| | **Natriumchlorit** | **Citronensäure** | **Natriumlaurylsulfat** |
| **Beispiel Nr.** | **[g]** | **[g]** | **[g]** |
| 23 | 0,075 | 0,052 | 0,050 |
| 24 | 0,149 | 0,103 | 0,050 |

**Tabelle 10: Konzentrationen von Chlorit, Citronensäure und Natriumlaurylsulfat in der hergestellten Lösung in den Beispielen 23 und 24.**

| **Beispiel Nr.** | **Konzentrationen in der Lösung** | | |
|---|---|---|---|
| | **Chlorit** | **Citronensäure** | **Natriumlaurylsulfat** |
| | **[ppm]** | **[ppm]** | **[ppm]** |
| 23 | 100 | 104 | 100 |
| 24 | 200 | 206 | 100 |

### BEZUGSZEICHENLISTE

- 100: Vorrichtung
- 101: Anschlusselement
- 102: Dorn
- 103: erstes Reservoir
- 104: antiseptisches Oxidationsmittel
- 105: Ausgang des ersten Reservoirs
- 106: Anschlussfilter
- 107: Fluidikelement
- 108: Kolben
- 109: erstes Verbindungselement
- 110: Düse
- 111: erste Durchführung
- 112: zweite Durchführung
- 113: zweites Verbindungselement
- 114: Verschluss
- 115: Hohlraum des Dorns
- 116: Halterung für Filter
- 119: erster Verschluss
- 120: Abgabeelement
- 123: zweites Reservoir
- 124: Tensid
- 126: Zwischenfilter
- 129: zweiter Verschluss
- 150: Adapterteil
- 160: Mischerteil
- 200: Behälter
- 201: Septum
- 202: Flüssigkeit
- 203: Behälterverschluss

## Patentansprüche

1. Vorrichtung (100) zur Herstellung einer antiseptischen Wundspüllösung, aufweisend ein antiseptisches Oxidationsmittel (104) und ein Tensid (124).

2. Vorrichtung nach Anspruch 1, wobei das antiseptische Oxidationsmittel und das Tensid in der Vorrichtung getrennt voneinander vorliegen.

3. Vorrichtung nach einem der vorangehenden Ansprüche, wobei das antiseptische Oxidationsmittel und/oder das Tensid als Feststoff vorliegt.

4. Vorrichtung nach einem der vorangehenden Ansprüche, welche weiterhin ein erstes Reservoir (103) und ein davon getrenntes zweites Reservoir (123) aufweist, wobei das antiseptische Oxidationsmittel in dem ersten Reservoir (103) angeordnet ist, und das Tensid in dem zweiten Reservoir (123) angeordnet ist.

5. Vorrichtung nach einem der vorangehenden Ansprüche, wobei die Vorrichtung weiterhin einen Zwischenfilter (106) aufweist, der das erste Reservoir (103) fluidleitend mit dem zweiten Reservoir (123) verbindet, wobei der Zwischenfilter bevorzugt für das antiseptische Oxidationsmittel und/oder das Tensid nur in flüssigem Zustand durchlässig ist.

6. Vorrichtung nach einem der vorangehenden Ansprüche, wobei die Vorrichtung weiterhin ein Anschlusselement (101) aufweist, wobei das Anschlusselement eingerichtet ist, eine fluidleitende Verbindung mit einem Behälter (200) herzustellen.

7. Vorrichtung nach Anspruch 6, wobei das antiseptische Oxidationsmittel und/oder das Tensid als Feststoff vorliegt, und wobei das Anschlusselement (101) einen Anschlussfilter (106) aufweist, welcher eingerichtet ist, das antiseptische Oxidationsmittel und/oder das Tensid als Feststoff in dem ersten Reservoir (103) und/oder dem zweiten Reservoir (123) zurückzuhalten.

8. Vorrichtung nach Anspruch 6 oder 7, weiterhin aufweisend ein Abgabeelement (120), wobei das Abgabeelement bevorzugt eingerichtet ist, eine aus dem antiseptischen Oxidationsmittel und dem Tensid hergestellte desinfizierende Wundspüllösung aus der Vorrichtung abzugeben.

9. Vorrichtung nach einem der vorangehenden Ansprüche, welche weiterhin einen Dorn (102) aufweist, der zum Durstechen eines Septums eines Behälters (200) eingerichtet ist, wobei der Dorn bevorzugt einen hohlen Innenraum (115) aufweist, der zur Aufnahme einer Flüssigkeit (202) aus dem Behälter (200) in das erste Reservoir (103) und das zweite Reservoir (123) eingerichtet ist.

10. Vorrichtung nach einem der vorangehenden Ansprüche, welche weiterhin ein Fluidikelement (107) aufweist, das eingerichtet und ausgebildet ist, eine Flüssigkeit in die Vorrichtung zu fördern.

11. Vorrichtung nach einem der Ansprüche 4 bis 10, wobei die Vorrichtung ein Adapterteil (150) und ein Mischerteil (160) aufweist, wobei das Adapterteil zum Anschluss an einen Behälter (200) eingerichtet ist, und wobei das Mischerteil zum Mischen des antiseptischen Oxidationsmittels (104) und des Tensids (124) mit einer Flüssigkeit aus dem Behälter (200) eingerichtet ist.

12. Vorrichtung nach einem der vorangehenden Ansprüche, wobei das antiseptische Oxidationsmittel ausgewählt ist aus der Gruppe bestehend aus Chlorit, Hypochlorit und Peroxid, weiter bevorzugt ausgewählt aus der Gruppe bestehend aus Calciumhypochlorit, Magnesiumhypochlorit, Natriumchlorit, Natriumhypochlorit, Natriumperoxid und Calciumperoxid.

13. Vorrichtung nach einem der vorangehenden Ansprüche, wobei das Tensid ein Fettalkoholsulfat und/oder ein quartäres Ammoniumsalz umfasst; wobei das Tensid weiter bevorzugt Natriumdodecylsulfat und/oder Benzalkoniumchlorid umfasst.

14. Vorrichtung nach einem der vorangehenden Ansprüche, welche weiterhin einen pH-Regulator aufweist, wobei der pH-Regulator ausgewählt ist aus der Gruppe bestehend aus Citronensäure, Citrat, Glucuronsäure, Gluconat, Natriumhydrogensulfat, und Natriumhydrogencarbonat.

15. Verfahren zur Herstellung einer antiseptischen Wundspüllösung mithilfe einer Vorrichtung nach einem der vorangehenden Ansprüche, wobei das Verfahren die folgenden Schritte aufweist,
a) gegebenenfalls Auflösen oder Verdünnen des antiseptischen Oxidationsmittel und/oder des Tensid mithilfe einer medizinisch verträglichen Flüssigkeit mithilfe der Vorrichtung,
b) Mischen des antiseptischen Oxidationsmittel mit dem Tensid mithilfe der Vorrichtung, und dadurch Erhalt einer antiseptischen Wundspüllösung.

16. Verfahren nach Anspruch 15, wobei die Konzentration des antiseptischen Oxidationsmittels in der hergestellten Wundspüllösung 100 ppm bis 4000 ppm (Masse/Masse) beträgt, und/oder die Konzentration des Tensids in der hergestellten Wundspüllösung 25 ppm bis 2000 ppm (Masse/Masse) beträgt.

17. Verfahren nach einem der Ansprüche 15 oder 16, wobei der pH-Wert der hergestellten Wundspüllösung 4 bis 8, bevorzugt 5 bis 7 beträgt.

18. Medizinische Wundspüllösung zur Anwendung in einem Verfahren für die Prophylaxe oder Behandlung einer Wundinfektion, wobei die medizinische Wundspüllösung ein antiseptisches Oxidationsmittel und ein Tensid umfasst.

## Geänderte Patentansprüche

### Geänderte Patentansprüche gemäss Regel 137(2) EPÜ.

1. Vorrichtung (100) zur Herstellung einer antiseptischen Wundspüllösung, aufweisend ein antiseptisches Oxidationsmittel (104) und ein Tensid (124), und weiterhin ein erstes Reservoir (103) und ein davon getrenntes zweites Reservoir (123), wobei das antiseptische Oxidationsmittel in dem ersten Reservoir (103) angeordnet ist, und das Tensid in dem zweiten Reservoir (123) angeordnet ist,
wobei die Vorrichtung weiterhin einen Zwischenfilter (106) aufweist, der das erste Reservoir (103) fluidleitend mit dem zweiten Reservoir (123) verbindet.

2. Vorichtung nach Anspruch 1, wobei der Zwischenfilter für das antiseptische Oxidationsmittel und/oder das Tensid nur in flüssigem Zustand durchlässig ist.

3. Vorrichtung nach einem der vorangehenden Ansprüche, wobei die Vorrichtung weiterhin ein Anschlusselement (101) aufweist, wobei das Anschlusselement eingerichtet ist, eine fluidleitende Verbindung mit einem Behälter (200) herzustellen.

4. Vorrichtung nach Anspruch 3, wobei das antiseptische Oxidationsmittel und/oder das Tensid als Feststoff vorliegt, und wobei das Anschlusselement (101) einen Anschlussfilter (106) aufweist, welcher eingerichtet ist, das antiseptische Oxidationsmittel und/oder das Tensid als Feststoff in dem ersten Reservoir (103) und/oder dem zweiten Reservoir (123) zurückzuhalten.

5. Vorrichtung nach Anspruch 4 oder 5, weiterhin aufweisend ein Abgabeelement (120), wobei das Abgabeelement bevorzugt eingerichtet ist, eine aus dem antiseptischen Oxidationsmittel und dem Tensid hergestellte desinfizierende Wundspüllösung aus der Vorrichtung abzugeben.

6. Vorrichtung nach einem der vorangehenden Ansprüche, welche weiterhin einen Dorn (102) aufweist, der zum Durstechen eines Septums eines Behälters (200) eingerichtet ist, wobei der Dorn bevorzugt einen hohlen Innenraum (115) aufweist, der zur Aufnahme einer Flüssigkeit (202) aus dem Behälter (200) in das erste Reservoir (103) und das zweite Reservoir (123) eingerichtet ist.

7. Vorrichtung nach einem der vorangehenden Ansprüche, welche weiterhin ein Fluidikelement (107) aufweist, das eingerichtet und ausgebildet ist, eine Flüssigkeit in die Vorrichtung zu fördern.

8. Vorrichtung nach einem der Ansprüche 1 bis 7, wobei die Vorrichtung ein Adapterteil (150) und ein Mischerteil (160) aufweist, wobei das Adapterteil zum Anschluss an einen Behälter (200) eingerichtet ist, und wobei das Mischerteil zum Mischen des antiseptischen Oxidationsmittels (104) und des Tensids (124) mit einer Flüssigkeit aus dem Behälter (200) eingerichtet ist.

9. Vorrichtung nach einem der vorangehenden Ansprüche, wobei das antiseptische Oxidationsmittel ausgewählt ist aus der Gruppe bestehend aus Chlorit, Hypochlorit und Peroxid, weiter bevorzugt ausgewählt aus der Gruppe bestehend aus Calciumhypochlorit, Magnesiumhypochlorit, Natriumchlorit, Natriumhypochlorit, Natriumperoxid und Calciumperoxid.

10. Vorrichtung nach einem der vorangehenden Ansprüche, wobei das Tensid ein Fettalkoholsulfat und/oder ein quartäres Ammoniumsalz umfasst; wobei das Tensid weiter bevorzugt Natriumdodecylsulfat und/oder Benzalkoniumchlorid umfasst.

11. Vorrichtung nach einem der vorangehenden Ansprüche, welche weiterhin einen pH-Regulator aufweist, wobei der pH-Regulator ausgewählt ist aus der Gruppe bestehend aus Citronensäure, Citrat, Glucuronsäure, Gluconat, Natriumhydrogensulfat, und Natriumhydrogencarbonat.

12. Verfahren zur Herstellung einer antiseptischen Wundspüllösung mithilfe einer Vorrichtung nach einem der vorangehenden Ansprüche, wobei das Verfahren die folgenden Schritte aufweist,
a) gegebenenfalls Auflösen oder Verdünnen des antiseptischen Oxidationsmittel und/oder des Tensid mithilfe einer medizinisch verträglichen Flüssigkeit mithilfe der Vorrichtung,
b) Mischen des antiseptischen Oxidationsmittel mit dem Tensid mithilfe der Vorrichtung, und dadurch Erhalt einer antiseptischen Wundspüllösung.

13. Verfahren nach Anspruch 12, wobei die Konzentration des antiseptischen Oxidationsmittels in der hergestellten Wundspüllösung 100 ppm bis 4000 ppm (Masse/Masse) beträgt, und/oder die Konzentration des Tensids in der hergestellten Wundspüllösung 25 ppm bis 2000 ppm (Masse/Masse) beträgt.

14. Verfahren nach einem der Ansprüche 12oder 13, wobei der pH-Wert der hergestellten Wundspüllösung 4 bis 8, bevorzugt 5 bis 7 beträgt.

15. Medizinische Wundspüllösung zur Anwendung in einem Verfahren für die Prophylaxe oder Behandlung einer Wundinfektion, wobei die medizinische Wundspüllösung ein antiseptisches Oxidationsmittel und ein Tensid umfasst.
